# EUROPEAN PATENT SPECIFICATION

(11) **EP 2 027 081 B1**
(45) Date of publication and mention of the grant of the patent: **03.05.2017**
(21) Application number: 07759457.0
(22) Date of filing: 27.03.2007
(51) Int. Cl.: C07C 59/56, C07C 59/86, C07C 59/90, C07C 69/732, C07C 69/738, C07D 333/40, A61K 31/5575, A61P 27/06, C07D 333/22, C07D 333/38

(54) **THERAPEUTIC PROSTAGLANDIN COMPOUNDS FOR THE TREATMENT OF GLAUCOMA**
THERAPEUTISCHE PROSTAGLANDIN-VERBINDUNGEN ZUR BEHANDLUNG VON GLAUKOMEN
COMPOSES THERAPEUTIQUES A BASE DE PROSTAGLANDINES POUR LE TRAITEMENT DU GLAUCOME

(30) Priority: 04.04.2006 US 744236 P
(43) Date of publication of application: 25.02.2009
(73) Proprietor: ALLERGAN, INC., Irvine, CA 92612 (US)
(72) Inventor: DONDE, Yariv, Dana Point, CA 92629 (US); NGUYEN, Jeremiah, H., La Puente, CA 91744 (US); HOLOBOSKI, Mark, Laguna Niguel, CA 92677 (US); POSNER, Mari, F., Laguna Niguel, CA 92677 (US); BURK, Robert, M., Laguna Beach, CA 92651 (US)
(74) Representative: Hoffmann Eitle
(86) International application number: PCT/US2007/065010
(87) International publication number: WO 2007/115020

(56) References cited:
- WO-A-95/19964
- WO-A1-2005/061449
- DATABASE CA [Online] CHEMICAL ABSTRACTS SERVICE, COLUMBUS, OHIO, US; KONDRATENKOVA, E. M. ET AL: "Synthesis and biological activity of 11-deoxy analogues of PGE and PGF" XP002451149 retrieved from STN Database accession no. 2000:496782 & VESTSI NATSYYANAL'NAI AKADEMII NAVUK BELARUSI, SERYYA KHIMICHNYKH NAVUK , (4), 58-62 CODEN: VNBNFX; ISSN: 1561-8331, 1999,
- DATABASE CA [Online] CHEMICAL ABSTRACTS SERVICE, COLUMBUS, OHIO, US; KUZ'MITSKII, B. B. ET AL: "Biological activity of some new synthetic prostaglandins of the 11-deoxy E series" XP002451150 retrieved from STN Database accession no. 1988:198903 & VESTSI AKADEMII NAVUK BSSR, SERYYA KHIMICHNYKH NAVUK , (1), 56-9 CODEN: VBSKAK; ISSN: 0002-3590, 1988,

## Description

Glaucoma is a disease of the eye characterized by increased intraocular pressure. If left untreated, it can lead to loss of visual field or blindness. Treatment with medications to reduce intraocular pressure is one way of preventing the development of glaucoma in people with elevated intraocular pressure, or of preventing further loss of visual field in people with glaucoma.

Certain eicosanoids and their derivatives are currently commercially available for use in controlling intraocular pressure. Eicosanoids and derivatives include numerous biologically important compounds such as prostaglandins and their derivatives. Prostaglandins can be described as derivatives of prostanoic acid which have the following structural formula:

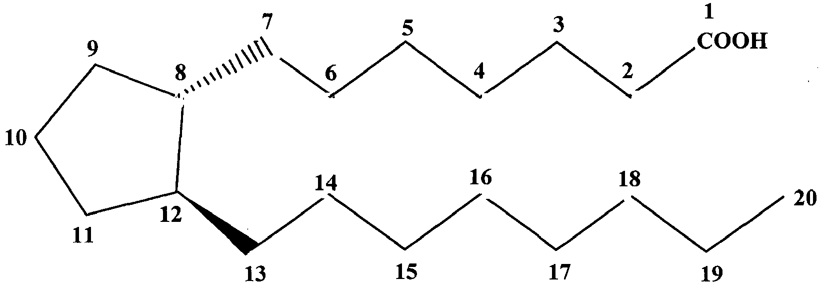

Various types of prostaglandins are known, depending on the structure and substituents carried on the alicyclic ring of the prostanoic acid skeleton. Further classification is based on the number of unsaturated bonds in the side chain indicated by numerical subscripts after the generic type of prostaglandin [e.g. prostaglandin E₁ (PGE₁), prostaglandin E₂ (PGE₂)], and on the configuration of the substituents on the alicyclic ring indicated by α or β [e.g. prostaglandin F_{2α} (PGF_{2ß})].

WO 2005/061449 relates to a compound selected from (1R,2S)-2[4-1(S)-hydroxyhexyl)phenyl]-5-oxo-cyclopentaneheptanoic acid [RSS] and (1R,2S)-2[4-1(R)-hydroxyhexyl)phenyl]-5-oxo-cyclopentaneheptanoic acid [RSR] or a salt, solvate, chemically protected form or prodrug thereof, and its use in treating conditions alleviated by agonism of an EP2 receptor.

WO 1995019964 A1 relates to (±) trans- 2-[-4(1-hydroxyhexyl) phenyl]-5-oxocyclopentaneheptanoicacid, and ester, unsaturated, and prostaglandin derivatives thereof. More particularly, this patent application relates to compounds of formula (I), wherein the wavy bonds indicate the α or p configuration, the dashed bond represents either a single or double bond, which double bond may be a cis or trans double bond and R is a saturated or unsaturated acyclic hydrocarbon group having from 1 to about 20 carbon atoms, or -(CH₂)ₘR1- wherein m is 0-10, and R1 is an aliphatic ring having from about 3 to about 7 carbon atoms, or an aryl or heteroaryl ring having from about 4 to about 10 carbon atoms and wherein the heteroatom is selected from the group consisting of N, O and S. According to this disclosure, these compounds are potent ocular hypotensives, and are particularly suitable for the management of glaucoma.

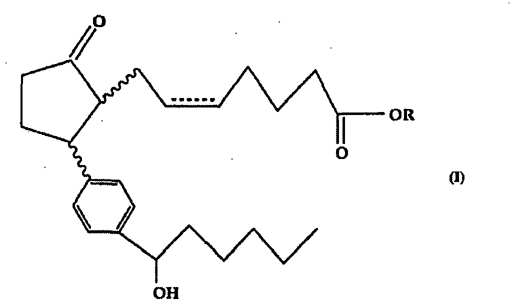

The invention is defined in the claims as a compound having the following structure or a pharmaceutically acceptable salt thereof: 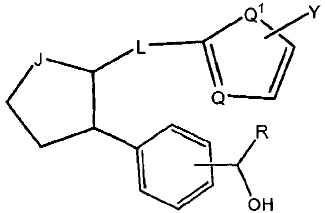
wherein Y is an organic acid functional group, or an amide or ester thereof comprising up to 14 carbon atoms; or Y is hydroxymethyl or an ether thereof comprising up to 14 carbon atoms; or Y is a tetrazolyl functional group;
L is linear C₃H₆, C₂H₄O, or C₂H₄S;
Q is CH, S, N, or O;
Q¹ is S, N, or O;
R is hydrogen or C₁₋₁₀ hydrocarbyl, and J is C=O, CHOH, CHF, CHCl, CHBr, or CHCN.

These compounds are useful in reducing intraocular pressure, thus preventing or delaying glaucoma in those with ocular hypertension, and preventing or delaying further vision loss in those with glaucoma.

"Bioisosteres are substituents or groups that have chemical or physical similarities, and which produce broadly similar biological properties." Silverman, Richard B., The Organic Chemistry of Drug Design and Drug Action, 2nd Edition, Amsterdam: Elsevier Academic Press, 2004, p. 29.

Organic acid functional groups are bioisoteres of carboxylic acids. An organic acid functional group is an acidic functional group on an organic molecule. Organic acid functional groups may comprise an oxide of carbon, sulfur, or phosphorous. Thus, in certain compounds Y is a carboxylic acid, sulfonic acid, or phosphonic acid functional group.

Additionally, an amide or ester of one of the organic acids shown above comprising up to 14 carbon atoms is also contemplated. In an ester, a hydrocarbyl moiety replaces a hydrogen atom of an acid such as in a carboxylic acid ester, e.g. CO₂Me, CO₂Et, etc.

In an amide, an amine group replaces an OH of the acid. Examples of amides include CON(R²)₂, CON(OR²)R², CON(CH₂CH₂OH)₂, and CONH(CH₂CH₂OH) where R² is independently H, C₁-C₆ alkyl, phenyl, or biphenyl. Moieties such as CONHSO₂R² are also amides of the carboxylic acid notwithstanding the fact that they may also be considered to be amides of the sulfonic acid R²-SO₃H. The following amides are also specifically contemplated, CONSO₂-biphenyl, CONSO₂-phenyl, CONSO₂-heteroaryl, and CONSO₂-naphthyl. The biphenyl, phenyl, heteroaryl, or naphthyl may be substituted or unsubstituted.

Han et. al. (Biorganic & Medicinal Chemistry Letters 15 (2005) 3487-3490) has recently shown that the groups shown below are suitable bioisosteres for a carboxylic acid. The activity of compounds with these groups in inhibiting HCV NS3 protease was comparable to or superior to similar compounds where the group is replaced by CO₂H. Thus, Y could be any group depicted below.

### Carboxylic acid bioisosteres according to Han et. al.

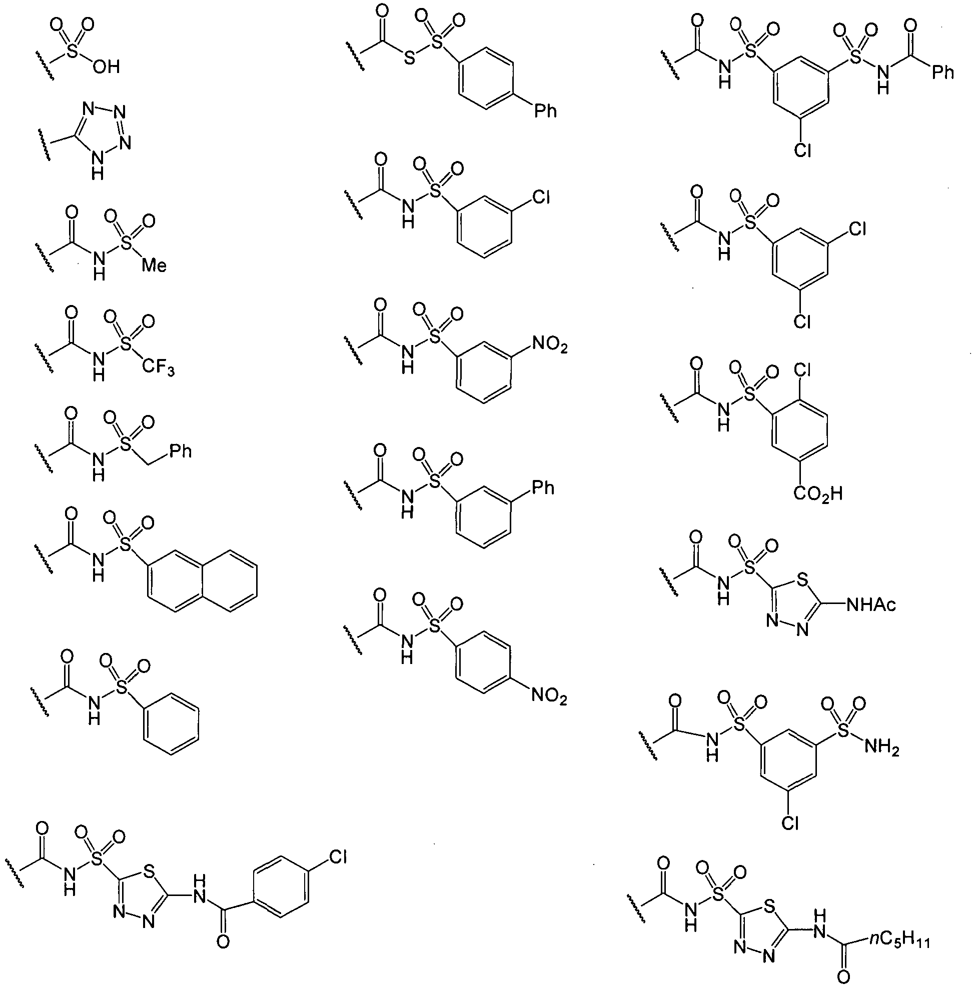

Y may also be hydroxymethyl or an ether thereof comprising up to 14 carbon atoms. An ether is a functional group wherein a hydrogen of an hydroxyl is replaced by carbon, e.g., Y is CH₂OCH₃, CH₂OCH₂CH₃, etc. These groups are also bioisosteres of a carboxylic acid.

"Up to 14 carbon atoms" means that the entire Y moiety, including the carbonyl carbon of a carboxylic acid ester or amide, and both carbon atoms in the -CH₂O-C of an ether has 0,1, 2, 3, 4, 5, 6, 7, 8, 9, 10, 11, 12, 13, or 14 carbon atoms.

Finally, Y may be a tetrazolyl functional group.

Examples of compounds having the identified Y are depicted below. In these examples R is H or hydrocarbyl, subject to the constraints defined herein. Each structure below represents a specific embodiment which is individually contemplated, as well as pharmaceutically acceptable salts of compounds which are represented by the structures. However, other examples are possible which may not fall within the scope of the structures shown below.

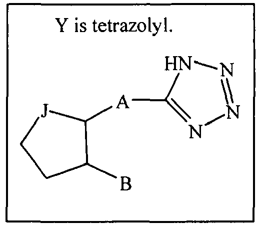

| **Organic Acids** | **Esters** | **Amides** |
|---|---|---|
| 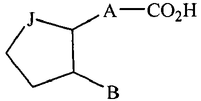 | 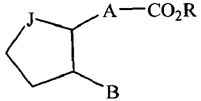 | 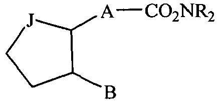 |
| Carboxylic Acid | Carboxylic Acid Ester | Carboxylic Acid Amide |
| 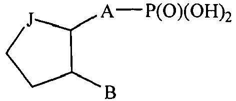 | 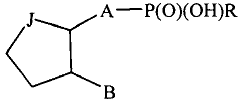 | 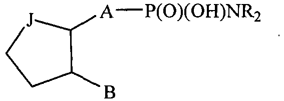 |
| Phosponic Acid | Phosphonic Acid Ester | Phosphonic Acid Amide |
| 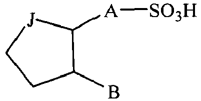 | 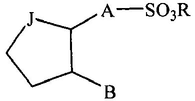 | 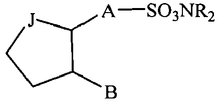 |
| Sulfonic Acid | Sulfonic Acid Ester | Sulfonic Acid Amide |
| 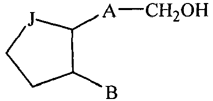 | 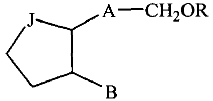 | |
| Y is hydroxymethyl | Ether | |

A tetrazolyl functional group is another bioisostere of a carboxylic acid. An unsubstituted tetrazolyl functional group has two tautomeric forms, which can rapidly interconvert in aqueous or biological media, and are thus equivalent to one another. These tautomers are shown below. 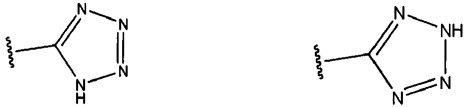 Additionally, if R² is C₁-C₆ alkyl, phenyl, or biphenyl, other isomeric forms of the tetrazolyl functional group such as the one shown below are also possible, unsubstituted and hydrocarbyl substituted tetrazolyl up to C₁₂ are considered to be within the scope of the term "tetrazolyl."

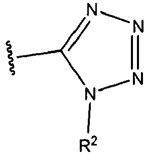

In one embodiment, Y is CO₂R², CON(R²)₂, CON(OR²)R², CON(CH₂CH₂OH)₂, CONH(CH₂CH₂OH), CH₂OH, P(O)(OH)₂, CONHSO₂R², SO₂N(R²)₂, SO₂NHR², 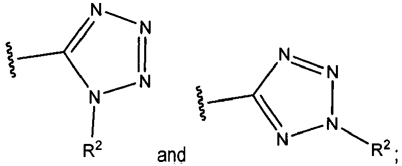 wherein R² is independently H, C₁-C₆ alkyl, unsubstituted phenyl, or unsubstituted biphenyl.

According to Silverman (p. 30), the moieties shown below are also bioisosteres of a carboxylic acid.

### Carboxylic acid bioisosteres according to Silverman

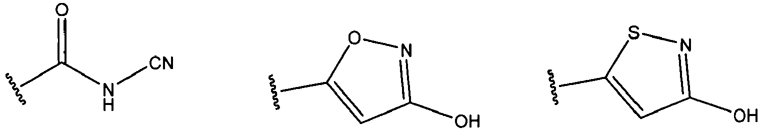

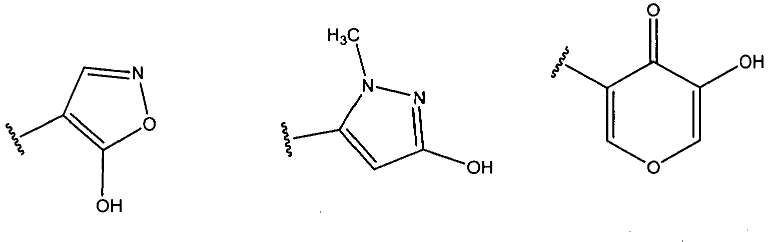

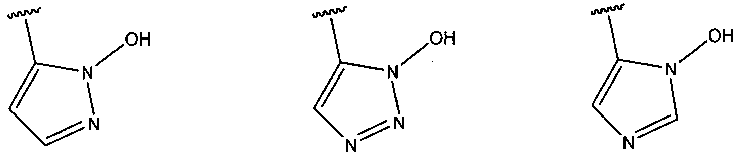

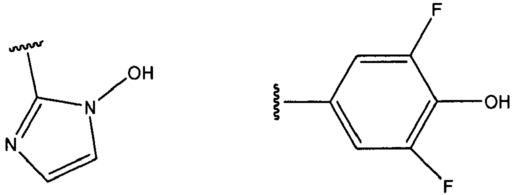

Orlek et al. (J. Med. Chem. 1991, 34, 2726-2735) described oxadiazoles as suitable bioisosteres for a carboxylic acid. These ester replacements were shown to be potent muscarinic agonists having improved metabolic stability. Oxadiazoles were also described by Anderson et al. (Eur. J. Med. Chem. 1996, 31, 417-425) as carboxamide replacements having improved in vivo efficacy at the benzodiazepine receptor.

### Carboxylic acid bioisosteres according to Orlek et. al.

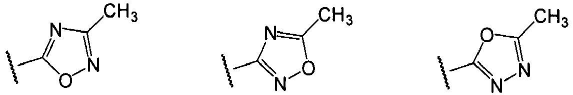

Kohara et al. (J. Med. Chem. 1996, 39, 5228-5235) described acidic heterocycles as suitable bioisosteres for a tetrazole. These carboxylic acid replacements were shown to be potent angiotensin II receptor antagonists having improved metabolic stability.

### Tetrazole bioisosteres according to Kohara et. al.

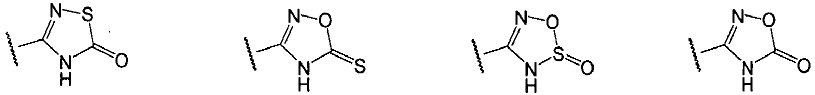

Drysdale et al. (J. Med. Chem. 1992, 35, 2573-2581) have described carboxylic acid mimics of non-peptide CCK-B receptor antagonists. The binding affinities of many of the bioisosteres are similar to the parent carboxylic acid.

### Carboxylic acid bioisosteres according to Drysdale et. al.

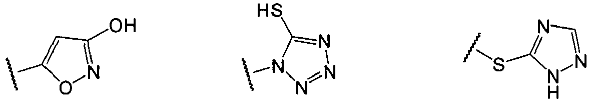

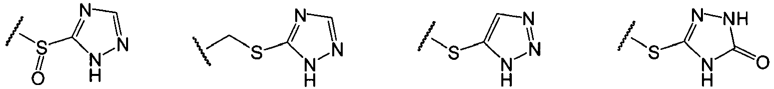

In some embodiments of the invention, L and the heterocyclic ring attached to it have one of the following structures, where Y is attached to the heteroaromatic ring.

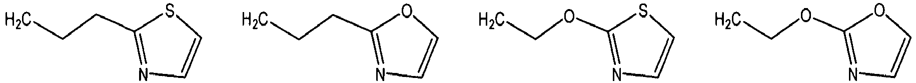

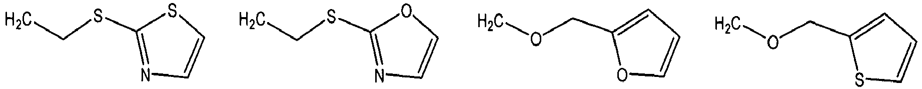

In another embodiment L and the heterocyclic ring attached to it are -CH₂-O-CH₂-Ar-, wherein Ar is 2,5-interthienylene.

In another embodiment L and the heterocyclic ring attached to it are -CH₂-O-CH₂-Ar-, wherein Ar is 2,5-interfurylene.

In another embodiment L and the heterocyclic ring attached to it are 2-(2-ethylthio)thiazol-4-yl.

In another embodiment L and the heterocyclic ring attached to it are 2-(3-propyl)thiazol-5-yl.

In another embodiment L and the heterocyclic ring attached to it are 5-(methoxymethyl)furan-2-yl.

In another embodiment L and the heterocyclic ring attached to it are 5-(methoxymethyl)thiophen-2-yl.

In another embodiment L and the heterocyclic ring attached to it are 5-(3-propyl)furan-2-yl.

In another embodiment L and the heterocyclic ring attached to it are 5-(3-propyl)thiophen-2-yl.

Compounds according to the each of the structures depicted below, and pharmaceutically acceptable salts thereof, are contemplated as individual embodiments of the invention, in which Y, J, and the phenyl substituent (identified in the structures below as 'B') correspond to the groups defined in the claim. In other words, each structure represents a different embodiment.

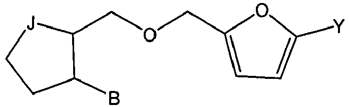

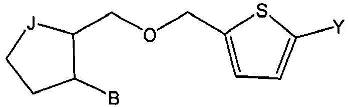

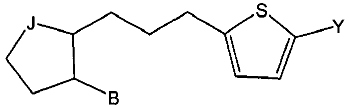

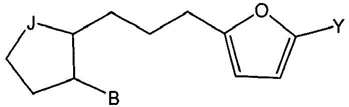

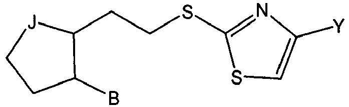

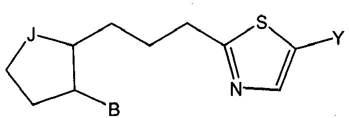

J is C=O, CHOH, CHF, CHCl, CHBr, or CHCN. Thus, each structure depicted below represents a compound embodiment which is individually contemplated, in which L and the heterocyclic ring attached to it (identified in the structures below as 'A'), the phenyl substituent (identified in the structures below as 'B'), and Y correspond to the groups defined in the claims. Pharmaceutically acceptable salts of compounds according to the structures below are also contemplated.

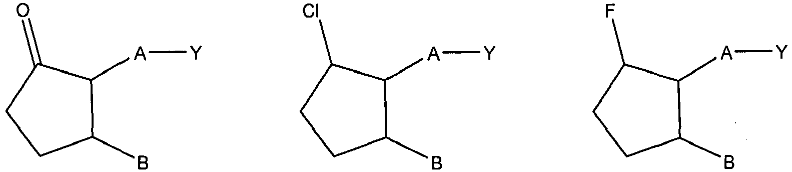

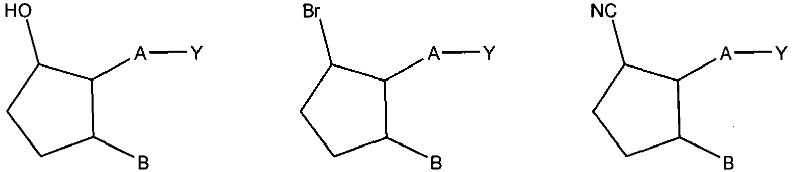

Aryl is an aromatic ring or ring system such as phenyl, naphthyl, biphenyl.

Heteroaryl is aryl having one or more N, O, or S atoms in the ring, i.e. one or more ring carbons are substituted by N, O, and/or S. Examples of heteroaryl include thienyl, pyridinyl, furyl, benzothienyl, benzofuryl, imidizololyl, indolyl.

A substituent of aryl or heteroaryl may have up to 20 non-hydrogen atoms each in any stable combination and as many hydrogen atoms as necessary, wherein the non-hydrogen atoms are C, N, O, S, P, F, Cl, Br, and/or I in any stable combination. However, the total number of non-hydrogen atoms on all of the substituents combined must also be 20 or less. A substituent must be sufficiently stable for the compound to be useful as described herein. In addition to the atoms listed above, a substituent may also have a metal cation or other stable cation having an atom not listed above if the substituent is acidic and the salt form is stable. For example, -OH may form an -O⁻Na⁺ salt or CO₂H may form a CO₂⁻K⁺ salt. Thus, a substituent may be:
hydrocarbyl, i.e. a moiety consisting of only carbon and hydrogen such as alkyl, alkenyl, alkynyl, including linear, branched or cyclic hydrocarbyl, and combinations thereof;
hydrocarbyloxy, meaning O-hydrocarbyl such as OCH₃, OCH₂CH₃, O-cyclohexyl, etc, up to 19 carbon atoms; other ether substituents such as CH₂OCH₃, (CH₂)₂OCH(CH₃)₂;
thioether substituents including S-hydrocarbyl and other thioether substituents;
hydroxyhydrocarbyl, meaning hydrocarbyl-OH such as CH₂OH, C(CH₃)₂OH, etc, up to 19 carbon atoms;
nitrogen substituents such as NO₂, CN, including
amino, such as NH₂, NH(CH₂CH₃OH), NHCH₃ up to 19 carbon atoms;
carbonyl substituents, such as CO₂H, ester, amide;
halogen, such as chloro, fluoro, bromo
fluorocarbyl, such as CF₃, CF₂CF₃, etc.;
phosphorous substituents, such as PO₃²⁻;
sulfur substituents, including S-hydrocarbyl, SH, SO₃H, SO₂-hydrocarbyl, SO₃-hydrocarbyl.

Substituted aryl or heteroaryl may have as many substituents as the ring or ring system will bear, and the substituents may be the same or different. Thus, for example, an aryl ring or a heteroaryl ring may be substituted with chloro and methyl; methyl, OH, and F; CN, NO₂, and ethyl; including any conceivable substituent or combination of substituent possible in light of this disclosure.

Subsituted aryl or substituted heteroaryl also includes a bicyclic or polycyclic ring system wherein one or more rings are aromatic and one or more rings are not. For example, indanonyl, indanyl, indanolyl, tetralonyl are substituted aryl. For this type of polycyclic ring system, an aromatic or heteroaromatic ring, not a non-aromatic ring, must be attached to the remainder of the molecule. In other words, in any structure depicting -B herein, where - is a bond, the bond is a direct bond to an aromatic ring.

In the compounds according to the invention is a substituted phenyl substituent as defined in the claims.

In one embodiment this phenyl substituent has no halogen atoms.

In another embodiment this phenyl substituent is 4-(1-hydroxy-2,2-dimethylpropyl)phenyl.

In another embodiment this phenyl substituent is 4-(1-hydroxy-2-methylpropyl)phenyl.

In another embodiment this phenyl substituent is 4-(1-hydroxybutyl)phenyl.

In another embodiment this phenyl substituent is 4-(1-hydroxyheptyl)phenyl.

In another embodiment this phenyl substituent is 4-(1-hydroxyhexyl)phenyl.

In another embodiment this phenyl substituent is 4-(1-hydroxypentyl)phenyl.

In another embodiment this phenyl substituent is 4-(1-hydroxypropyl)phenyl.

In another embodiment this phenyl substituent is 3-(hydroxy(1-propylcyclobutyl)methyl)phenyl.

In another embodiment this phenyl substituent is 4-(1-hydroxy-5,5-dimethylhexyl)phenyl.

In another embodiment this phenyl substituent is 4-(hydroxy(1-propylcyclobutyl)methyl)phenyl.

In another embodiment this phenyl substituent is 4-(1-hydroxy-2-phenylethyl)phenyl.

In another embodiment this phenyl substituent is 4-(1-hydroxy-3-phenylpropyl)phenyl.

In another embodiment this phenyl substituent is 4-(2-cyclohexyl-1-hydroxyethyl)phenyl.

In another embodiment this phenyl substituent is 4-(3-cyclohexyl-1-hydroxypropyl)phenyl.

In another embodiment this phenyl substituent is 4-(cyclohexyl(hydroxy)methyl)phenyl.

In another embodiment this phenyl substituent is 4-(hydroxy(phenyl)methyl)phenyl.

The compounds according to the invention have the following structures (wherein L and the heterocyclic ring attached to it (identified in the structures below as 'A') and J correspond to the groups defined in the claims) 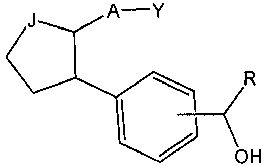 or a pharmaceutical salt thereof, or a prodrug thereof,
wherein R is hydrogen or C₁₋₁₀ hydrocarbyl.
Another embodiment is a compound according to the structure 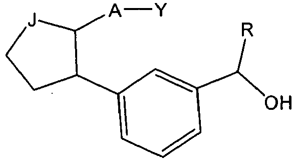 or a pharmaceutical salt thereof, or a prodrug thereof,
wherein R is hydrogen or C₁₋₁₀ hydrocarbyl.
Another embodiment is a compound according to the structure 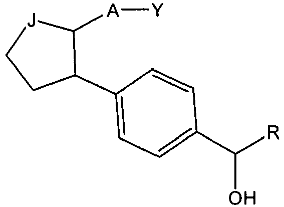 or a pharmaceutical salt thereof, or a prodrug thereof,
wherein R is hydrogen or C₁₋₁₀ hydrocarbyl.

In another embodiment R is n-pentyl.

The compound according to the present invention has the following structure, in which J and the phenyl substituent (identified in the structure below as 'B') are defined in the claims: 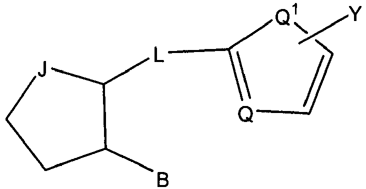 or a pharmaceutically acceptable salt thereof;
wherein L is linear C₃H₆, C₂H₄O, or C₂H₄S;
Q is CH, S, N, or O; and
Q¹ is S, N, or O.
That L is linear means that there are no branching groups and the connections to L are on opposite ends, i.e., the following groups are contemplated for L.

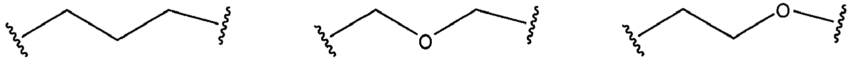

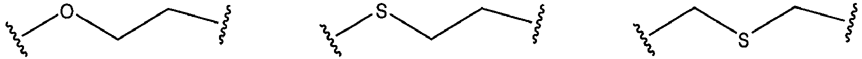

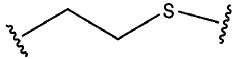

"C1-10" hydrocarbyl is hydrocarbyl having 1, 2, 3, 4, 5, 6, 7, 8, 9, or 10 carbon atoms.

Hydrocarbyl is a moiety consisting of only carbon and hydrogen, and includes alkenyl, alkynyl, and in some cases aryl, and combinations thereof.
Alkyl is hydrocarbyl having no double or triple bonds including:
linear alkyl such as methyl, ethyl, propyl, n-butyl, n-pentyl, n-hexyl;
branched alkyl such as isopropyl, branched butyl isomers (i.e. sec-butyl, tert-butyl, etc), branched pentyl isomers (i.e. isopentyl, etc), branched hexyl isomers, and higher branched alkyl fragments;
cycloalkyl such as cyclopropyl, cyclobutyl, cyclopentyl, cyclohexyl, cycloheptyl, etc.; and
alkyl fragments consisting of both cyclic and noncyclic components, whether linear or branched, which may be attached to the remainder of the molecule at any available position including terminal, internal, or ring carbon atoms.
Alkenyl is hydrocarbyl having one or more double bonds including
linear alkenyl, branched alkenyl, cyclic alkenyl, and combinations thereof in analogy to alkyl.
Alkynyl is hydrocarbyl having one or more triple bonds including linear alkynyl, branched alkynyl, cyclic alkynyl and combinations thereof in analogy to alkyl.
Aryl is an unsubstituted or substituted aromatic ring or ring system such as phenyl, naphthyl, biphenyl. Aryl may or may not be hydrocarbyl, depending upon whether it has substituents with heteroatoms.
Arylalkyl is alkyl which is substituted with aryl. In other words alkyl connects aryl to the remaining part of the molecule. Examples are -CH₂-Phenyl, -CH₂-CH₂-Phenyl. Arylalkyl may or may not be hydrocarbyl, depending upon whether it has substituents with heteroatoms.
Unconjugated dienes or polyenes have one or more double bonds which are not conjugated. They may be linear, branched, or cyclic, or a combination thereof.

Thus, each of the structures below is contemplated, in which L and the heterocyclic ring attached to it (identified in the structures below as 'A') and J correspond to the definition in the claims. These structures, or pharmaceutically acceptable salts thereof, individually represent a compound which is an embodiment contemplated herein. In other words, each structure represents a different embodiment.

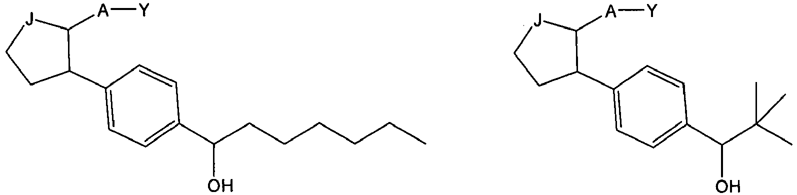

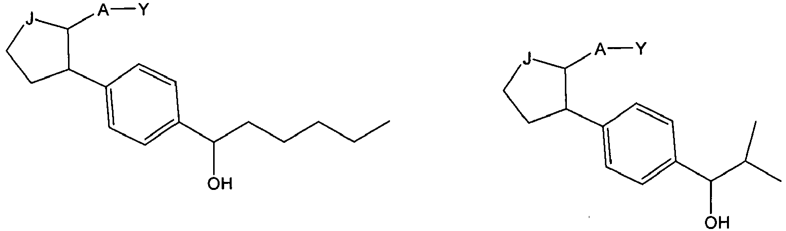

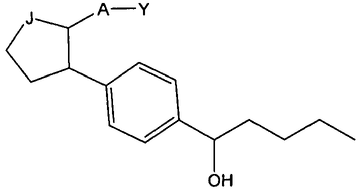

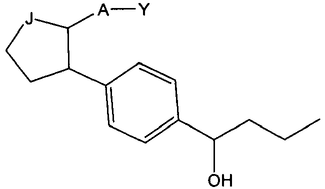

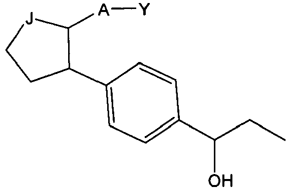

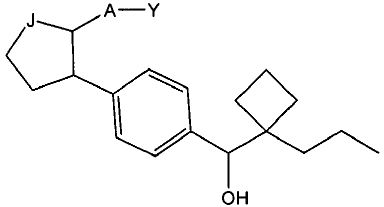

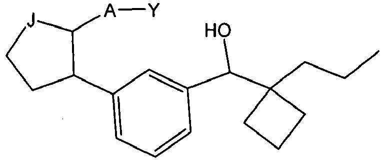

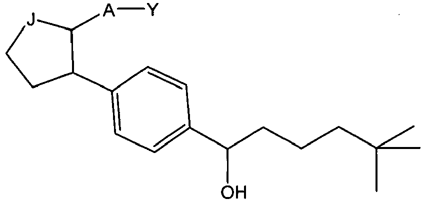

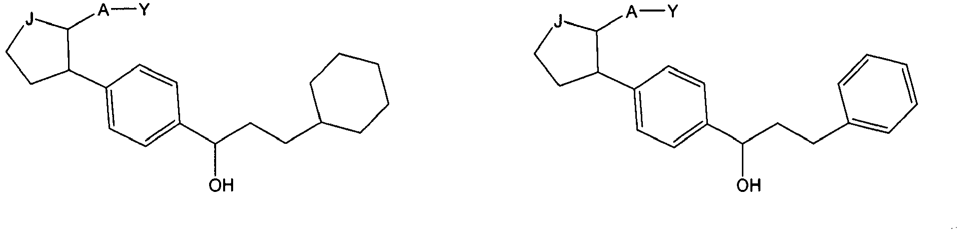

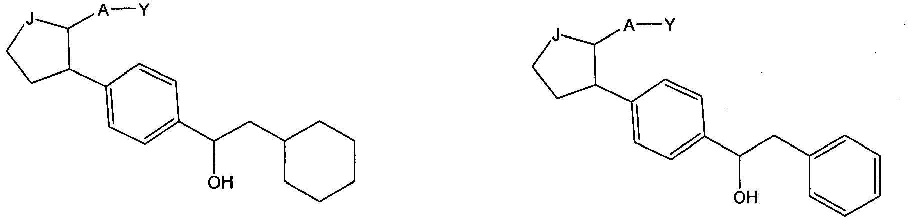

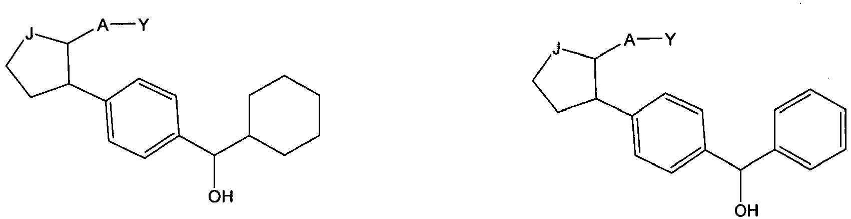

Hypothetical examples of useful compounds are shown below. 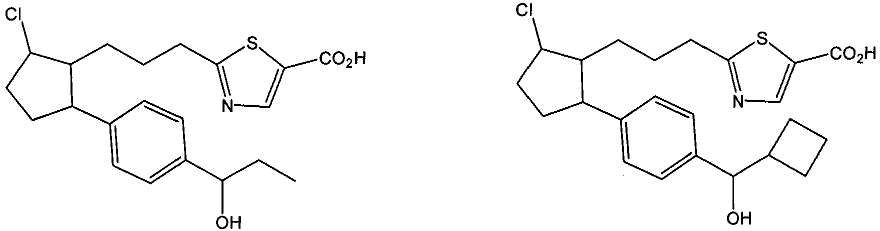 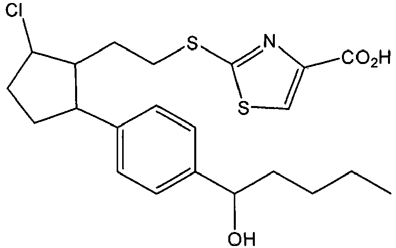 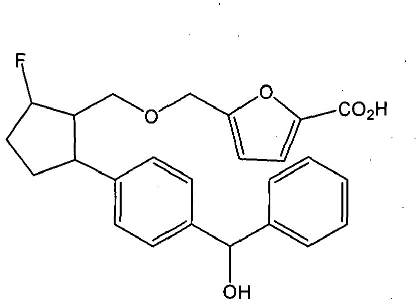 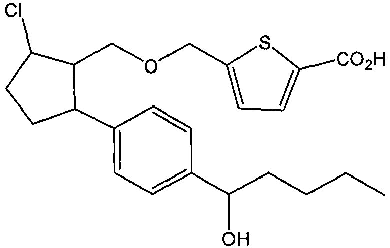 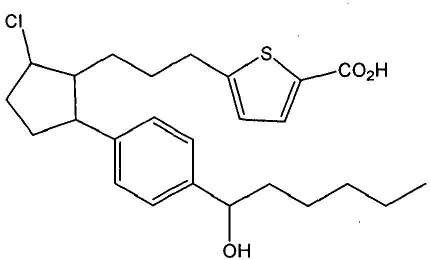 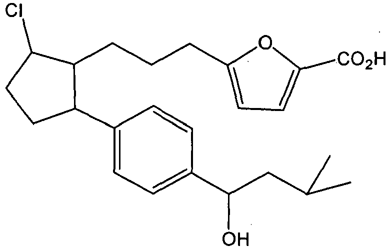 Other useful compounds include:
5-(3-{(1R,2R)-2-[4-(1-hydroxyhexyl)phenyl]-5-oxocyclopentyl}propyl)thiophene-2-carboxylic acid methyl ester,
5-(3-{(1R,2R)-2-[4-(1-hydroxyhexyl)phenyl]-5-oxocyclopentyl}propyl)thiophene-2-carboxylic acid,
5-(3-{(1R,2R)-2-[4-(1-hydroxyhexyl)phenyl]-5-oxocyclopentyl}propyl)thiophene-2-carboxylic acid isopropyl ester,
5-(3-{(1R,2R,5S)-2-Chloro-5-[4-(1-hydroxy-hexyl)-phenyl]-cyclopentyl}-propyl)-thiophene-2-carboxylic acid methyl ester,
5-(3-{(1R,2R,5S)-2-Chloro-5-[4-(1-hydroxy-hexyl)-phenyl]-cyclopentyl}-propyl)-thiophene-2-carboxylic acid ,
7-{(1R,2R,5S)-2-Chloro-5-[4-(cyclohexyl-hydroxy-methyl)-phenyl]-cyclopentyl}-heptanoic acid methyl ester, and
7-{(1R,2R,5S)-2-Chloro-5-[4-(cyclohexyl-hydroxy-methyl)-phenyl]-cyclopentyl}-heptanoic acid.

### Compound examples:

The following are hypothetical examples of useful compounds. Compound Examples 6, 7, 10 -15, 20, 21, 23, 30 - 33, 37, 38, 41, 45 do not form part of the invention.
**Compound Example 1.** A compound having a structure 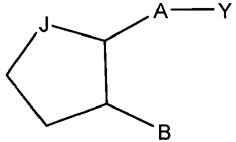 or a pharmaceutically acceptable salt thereof;
   wherein Y is an organic acid functional group, or an amide or ester thereof comprising up to 14 carbon atoms; or Y is hydroxymethyl or an ether thereof comprising up to 14 carbon atoms; or Y is a tetrazolyl functional group;
   A is L and the heterocyclic ring attached to it as defined in the claims;
   J is C=O, CHOH, CHF, CHCl, CHBr, or CHCN; and
   B is the phenyl substituent defined in the claims.
**Compound Example 2.** The compound according to compound example 1 wherein Y is selected from CO₂R², CON(R²)₂, CON(OR²)R², CON(CH₂CH₂OH)₂, CONH(CH₂CH₂OH), CH₂OH, P(O)(OH)₂, CONHSO₂R², SO₂N(R²)₂, SO₂NHR², 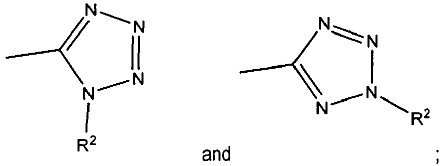 wherein R² is independently H, C₁-C₆ alkyl, unsubstituted phenyl, or unsubstituted biphenyl.
**Compound Example 3.** The compound according to compound example 1 or 2 wherein R is alkyl.
**Compound Example 4.** The compound according to compound example 1 or 2 wherein R is arylalkyl.
**Compound Example 5.** The compound according to any one of compound examples 1 to 4 having a structure 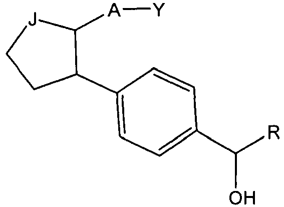 or a pharmaceutically acceptable salt thereof;
   R is hydrogen or C₁₋₁₀ hydrocarbyl.
**Compound Example 6.** The compound according to compound example 1 or 2 wherein L and the heterocyclic ring attached to it are (3-methylphenoxy)methyl.
**Compound Example 7.** The compound according to compound example 1 or 2 wherein L and the heterocyclic ring attached to it are (4-but-2-ynyloxy)methyl.
**Compound Example 8.** The compound according to compound example 1 or 2 wherein L and the heterocyclic ring attached to it are 2-(2-ethylthio)thiazol-4-yl.
**Compound Example 9.** The compound according to compound example 1 or 2 wherein L and the heterocyclic ring attached to it are 2-(3-propyl)thiazol-5-yl.
**Compound Example 10.** The compound according to compound example 1 or 2 wherein L and the heterocyclic ring attached to it are 3-methoxymethyl)phenyl.
**Compound Example 11.** The compound according to compound example 1 or 2 wherein L and the heterocyclic ring attached to it are 3-(3-propylphenyl).
**Compound Example 12.** The compound according to compound example 1 or 2 wherein L and the heterocyclic ring attached to it are 3-methylphenethyl.
**Compound Example 13.** The compound according to compound example 1 or 2 wherein L and the heterocyclic ring attached to it are 4-(2-ethyl)phenyl.
**Compound Example 14.** The compound according to compound example 1 or 2 wherein L and the heterocyclic ring attached to it are 4-phenethyl.
**Compound Example 15.** The compound according to compound example 1 or 2 wherein L and the heterocyclic ring attached to it are 4-methoxybutyl.
**Compound Example 16.** The compound according to compound example 1 or 2 wherein L and the heterocyclic ring attached to it are 5-(methoxymethyl)furan-2-yl.
**Compound Example 17.** The compound according to compound example 1 or 2 wherein L and the heterocyclic ring attached to it are 5-(methoxymethyl)thiophen-2-yl.
**Compound Example 18.** The compound according to compound example 1 or 2 wherein L and the heterocyclic ring attached to it are 5-(3-propyl)furan-2-yl.
**Compound Example 19.** The compound according to compound example 1 or 2 wherein L and the heterocyclic ring attached to it are 5-(3-propyl)thiophen-2-yl.
**Compound Example 20.** The compound according to compound example 1 or 2 wherein L and the heterocyclic ring attached to it are 6-hexyl.
**Compound Example 21.** The compound according to compound example 1 or 2 wherein L and the heterocyclic ring attached to it are (Z)-6-hex-4-enyl.
**Compound Example 22.** The compound according to any one of compound examples 1, 2, and 6-21 wherein the phenyl substituent is 4-(1-hydroxy-2,2-dimethylpropyl)phenyl.
**Compound Example 23.** The compound according to any one of compound examples 1, 2, and 6-21 wherein the phenyl substituent is 4-(1-hydroxy-2-methylpropan-2-yl)phenyl.
**Compound Example 24.** The compound according to any one of compound examples 1, 2, and 6-21 wherein the phenyl substituent is 4-(1-hydroxy-2-methylpropyl)phenyl.
**Compound Example 25.** The compound according to any one of compound examples 1, 2, and 6-21 wherein the phenyl substituent is 4-(1-hydroxybutyl)phenyl.
**Compound Example 26.** The compound according to any one of compound examples 1, 2, and 6-21 wherein the phenyl substituent is 4-(1-hydroxyheptyl)phenyl.
**Compound Example 27.** The compound according to any one of compound examples 1, 2, and 6-21 wherein the phenyl substituent is 4-(1-hydroxyhexyl)phenyl.
**Compound Example 28.** The compound according to any one of compound examples 1, 2, and 6-21 wherein the phenyl substituent is 4-(1-hydroxypentyl)phenyl.
**Compound Example 29.** The compound according to any one of compound examples 1, 2, and 6-21 wherein the phenyl substituent is 4-(1-hydroxypropyl)phenyl.
**Compound Example 30.** The compound according to any one of compound examples 1, 2, and 6-21 wherein the phenyl substituent is 4-(3-hydroxy-2-methylheptan-2-yl)phenyl.
**Compound Example 31.** The compound according to any one of compound examples 1, 2, and 6-21 wherein the phenyl substituent is 4-(3-hydroxy-2-methyloctan-2-yl)phenyl.
**Compound Example 32.** The compound according to any one of compound examples 1, 2, and 6-21 wherein the phenyl substituent is 1-hydroxy-2,3-dihydro-1H-inden-5-yl.
**Compound Example 33.** The compound according to any one of compound examples 1, 2, and 6-21 wherein the phenyl substituent is 2,3-dihydro-1H-inden-5-yl.
**Compound Example 34.** The compound according to any one of compound examples 1, 2, and 6-21 wherein the phenyl substituent is 3-(hydroxy(1-propylcyclobutyl)methyl)phenyl.
**Compound Example 35.** The compound according to any one of compound examples 1, 2, and 6-21 wherein the phenyl substituent is 4-(1-hydroxy-5,5-dimethylhexyl)phenyl.
**Compound Example 36.** The compound according to any one of compound examples 1, 2, and 6-21 wherein the phenyl substituent is 4-(hydroxy(1-propylcyclobutyl)methyl)phenyl.
**Compound Example 37.** The compound according to any one of compound examples 1, 2, and 6-21 wherein the phenyl substituent is 4-tert-butylphenyl.
**Compound Example 38.** The compound according to any one of compound examples 1, 2, and 6-21 wherein the phenyl substituent is 4-hexylphenyl.
**Compound Example 39.** The compound according to any one of compound examples 1, 2, and 6-21 wherein the phenyl substituent is 4-(1-hydroxy-2-phenylethyl)phenyl.
**Compound Example 40.** The compound according to any one of compound examples 1, 2, and 6-21 wherein the phenyl substituent is 4-(1-hydroxy-3-phenylpropyl)phenyl.
**Compound Example 41.** The compound according to any one of compound examples 1, 2, and 6-21 wherein the phenyl substituent is 4-(1-hydroxycyclobutyl)phenyl.
**Compound Example 42.** The compound according to any one of compound examples 1, 2, and 6-21 wherein the phenyl substituent is 4-(2-cyclohexyl-1-hydroxyethyl)phenyl.
**Compound Example 43.** The compound according to any one of compound examples 1, 2, and 6-21 wherein the phenyl substituent is 4-(3-cyclohexyl-1-hydroxypropyl)phenyl.
**Compound Example 44.** The compound according to any one of compound examples 1, 2, and 6-21 wherein the phenyl substituent is 4-(cyclohexyl(hydroxy)methyl)phenyl.
**Compound Example 45.** The compound according to any one of compound examples 1, 2, and 6-21 wherein the phenyl substituent is 4-(cyclohexylmethyl)phenyl.
**Compound Example 46.** The compound according to any one of compound examples 1, 2, and 6-21 wherein the phenyl substituent is 4-(hydroxy(phenyl)methyl)phenyl.
The following are hypothetical examples of compositions, kits, methods, uses, and medicaments employing the hypothetical compound examples.

*However, Compound Examples 6, 7, 10 -15, 20, 21, 23, 30 - 33, 37, 38, 41, and 45 do not form part of the invention.

### Composition Example:

A composition comprising a compound according to any one of compound examples 1 to 48*, wherein said composition is a liquid which is ophthalmically acceptable.

### Medicament Examples:

Use of a compound according to any one of compound examples 1 to 46* in the manufacture of a medicament for the treatment of glaucoma or ocular hypertension in a mammal.

A medicament comprising a compound according to any one of compound examples 1 to 46*, wherein said composition is a liquid which is ophthalmically acceptable.

### Method Example:

A compound according to any one of compound examples 1 to 46* for use in a method of treatment of glaucoma or ocular hypertension comprising administering the compound to a mammal.

### Kit Example:

A kit comprising a composition comprising compound according to any one of compound examples 1 to 46*, a container, and instructions for administration of said composition to a mammal for the treatment of glaucoma or ocular hypertension.

For the purposes of this disclosure, "treat," "treating," or "treatment" refer to the use of a compound, composition, therapeutically active agent, or drug in the diagnosis, cure, mitigation, treatment, or prevention of disease or other undesirable condition.

A person of ordinary skill in the art understands the meaning of the stereochemistry associated with the hatched wedge/solid wedge structural features. For example, an introductory organic chemistry textbook (Francis A. Carey, Organic Chemistry, New York: McGraw-Hill Book Company 1987, p. 63) states "a wedge indicates a bond coming from the plane of the paper toward the viewer" and the hatched wedge, indicated as a "dashed line", "represents a bond receding from the viewer."

A "pharmaceutically acceptable salt" is any salt that retains the activity of the parent compound and does not impart any additional deleterious or untoward effects on the subject to which it is administered and in the context in which it is administered compared to the parent compound. A pharmaceutically acceptable salt also refers to any salt which may form in vivo as a result of administration of an acid, another salt, or a prodrug which is converted into an acid or salt.

Pharmaceutically acceptable salts of acidic functional groups may be derived from organic or inorganic bases. The salt may comprise a mono or polyvalent ion. Of particular interest are the inorganic ions lithium, sodium, potassium, calcium, and magnesium. Organic salts may be made with amines, particularly ammonium salts such as mono-, di- and trialkyl amines or ethanol amines. Salts may also be formed with caffeine, tromethamine and similar molecules. Hydrochloric acid or some other pharmaceutically acceptable acid may form a salt with a compound that includes a basic group, such as an amine or a pyridine ring.

Those skilled in the art will readily understand that for administration or the manufacture of medicaments the compounds disclosed herein can be admixed with pharmaceutically acceptable excipients which per se are well known in the art. Specifically, a drug to be administered systemically, it may be confected as a powder, pill, tablet, or as a solution, emulsion, suspension, aerosol, syrup or elixir suitable for oral or parenteral administration or inhalation.

For solid dosage forms or medicaments, non-toxic solid carriers include pharmaceutical grades of mannitol, lactose, starch, magnesium stearate, sodium saccharin, the polyalkylene glycols, talcum, cellulose, glucose, sucrose and magnesium carbonate. The solid dosage forms may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the technique described in the U.S. Pat. Nos. 4,256,108; 4,166,452; and 4,265,874 to form osmotic therapeutic tablets for control release. Liquid pharmaceutically administrable dosage forms can, for example, comprise a solution or suspension of one or more of the presently useful compounds and optional pharmaceutical adjutants in a carrier, such as for example, water, saline, aqueous dextrose, glycerol, ethanol, to thereby form a solution or suspension. If desired, the pharmaceutical composition to be administered may also contain minor amounts of nontoxic auxiliary substances such as wetting or emulsifying agents, pH buffering agents. Typical examples of such auxiliary agents are sodium acetate, sorbitan monolaurate, triethanolamine, sodium acetate, triethanolamine oleate, etc. Actual methods of preparing such dosage forms are known, or will be apparent, to those skilled in this art; for example, see Remington's Pharmaceutical Sciences, Mack Publishing Company, Easton, Pa., 16th Edition, 1980. The composition of the formulation to be administered, in any event, contains a quantity of one or more of the presently useful compounds in an amount effective to provide the desired therapeutic effect.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol. In addition, if desired, the injectable pharmaceutical compositions to be administered may also contain minor amounts of non-toxic auxiliary substances such as wetting or emulsifying agents, pH buffering agents.

The amount of the presently useful compound or compounds administered is dependent on the therapeutic effect or effects desired, on the specific mammal being treated, on the severity and nature of the mammal's condition, on the manner of administration, on the potency and pharmacodynamics of the particular compound or compounds employed, and on the judgment of the prescribing physician. The therapeutically effective dosage of the presently useful compound or compounds may be in the range of about 0.5 or about 1 to about 100 mg/kg/day.

A liquid which is ophthalmically acceptable is formulated such that it can be administered topically to the eye. The comfort should be maximized as much as possible, although sometimes formulation considerations (e.g. drug stability) may necessitate less than optimal comfort. In the case that comfort cannot be maximized, the liquid should be formulated such that the liquid is tolerable to the patient for topical ophthalmic use. Additionally, an ophthalmically acceptable liquid should either be packaged for single use, or contain a preservative to prevent contamination over multiple uses.

For ophthalmic application, solutions or medicaments are often prepared using a physiological saline solution as a major vehicle. Ophthalmic solutions should preferably be maintained at a comfortable pH with an appropriate buffer system. The formulations may also contain conventional, pharmaceutically acceptable preservatives, stabilizers and surfactants.

Preservatives that may be used in the pharmaceutical compositions of the present invention include benzalkonium chloride, chlorobutanol, thimerosal, phenylmercuric acetate and phenylmercuric nitrate. A useful surfactant is, for example, Tween 80. Likewise, various useful vehicles may be used in the ophthalmic preparations of the present invention. These vehicles include polyvinyl alcohol, povidone, hydroxypropyl methyl cellulose, poloxamers, carboxymethyl cellulose, hydroxyethyl cellulose and purified water.

Tonicity adjustors may be added as needed or convenient. They include salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. Accordingly, buffers include acetate buffers, citrate buffers, phosphate buffers and borate buffers. Acids or bases may be used to adjust the pH of these formulations as needed.

In a similar vein, an ophthalmically acceptable antioxidant for use in the present invention includes sodium metabisulfite, sodium thiosulfate, acetylcysteine, butylated hydroxyanisole and butylated hydroxytoluene.

Other excipient components which may be included in the ophthalmic preparations are chelating agents. A useful chelating agent is edetate disodium, although other chelating agents may also be used in place or in conjunction with it.

The ingredients are usually used in the following amounts:

| **Ingredient** | **Amount (% w/v)** |
|---|---|
| active ingredient | about 0.001-5 |
| preservative | 0-0.10 |
| vehicle | 0-40 |
| tonicity adjustor | 1-10 |
| buffer | 0.01-10 |
| pH adjustor | q.s. pH 4.5-7.5 |
| antioxidant | as needed |
| surfactant | as needed |
| purified water | as needed to make 100% |

For topical use, creams, ointments, gels, solutions or suspensions, etc., containing the compound disclosed herein are employed. Topical formulations may generally be comprised of a pharmaceutical carrier, cosolvent, emulsifier, penetration enhancer, preservative system, and emollient.

The actual dose of the active compounds of the present invention depends on the specific compound, and on the condition to be treated; the selection of the appropriate dose is well within the knowledge of the skilled artisan.

The compounds disclosed herein are also useful in combination with other drugs useful for the treatment of glaucoma or other conditions.

For the treatment of glaucoma, combination treatment with the following classes of drugs are contemplated:
β-Blockers (or β-adrenergic antagonists) including carteolol, levobunolol, metiparanolol, timolol hemihydrate, timolol maleate, β1-selective antagonists such as betaxolol, or pharmaceutically acceptable salts thereof;
Adrenergic Agonists including
non-selective adrenergic agonists such as epinephrine borate, epinephrine hydrochloride, and dipivefrin, or pharmaceutically acceptable salts thereof; and
α₂-selective adrenergic agonists such as apraclonidine, brimonidine, or pharmaceutically acceptable salts thereof;
Carbonic Anhydrase Inhibitors including acetazolamide, dichlorphenamide, methazolamide, brinzolamide, dorzolamide, or pharmaceutically acceptable salts thereof;
Cholinergic Agonists including
direct acting cholinergic agonists such as carbachol, pilocarpine hydrochloride, pilocarbine nitrate, pilocarpine, or pharmaceutically acceptable salts thereof;
chlolinesterase inhibitors such as demecarium, echothiophate, physostigmine, or pharmaceutically acceptable salts thereof;
Glutamate Antagonists and other neuroprotective agents such as Ca²⁺ channel blockers such as memantine, amantadine, rimantadine, nitroglycerin, dextrophan, detromethorphan, CGS-19755, dihydropyridines, verapamil, emopamil, benzothiazepines, bepridil, diphenylbutylpiperidines, diphenylpiperazines, HOE 166 and related drugs, fluspirilene, eliprodil, ifenprodil, CP-101,606, tibalosine, 2309BT, and 840S, flunarizine, nicardipine, nifedimpine, nimodipine, barnidipine, verapamil, lidoflazine, prenylamine lactate, amiloride, or pharmaceutically acceptable salts thereof;
Prostamides such as bimatoprost, or pharmaceutically acceptable salts thereof; and
Prostaglandins including travoprost, UFO-21, chloprostenol, fluprostenol, 13,14-dihydro-chloprostenol, isopropyl unoprostone, latanoprost.
Cannabinoids including CB1 agonists such as WIN-55212-2 and CP-55940, or pharmaceutically acceptable salts thereof.

For treatment of diseases affecting the eye including glaucoma, these compounds can be administered topically, periocularly, intraocularly, or by any other effective means known in the art.

In addition to the treatment of glaucoma, prostaglandin EP₂ selective agonists are believed to have several medical uses. For example, U.S. Patent No. 6,437,146 teaches the use of prostaglandin EP₂ selective agonists "for treating or preventing inflammation and pain in joint and muscle (e.g., rheumatoid arthritis, rheumatoid spondylitis, osteoarthritis, gouty arthritis, juvenile arthritis, etc.), inflammatory skin condition (e.g., sunburn, burns, eczema, dermatitis, etc.), inflammatory eye condition (e.g., conjunctivitis, etc.), lung disorder in which inflammation is involved (e.g., asthma, bronchitis, pigeon fancier's disease, farmer's lung, etc.), condition of the gastrointestinal tract associated with inflammation (e.g., aphthous ulcer, Chrohn's disease, atrophic gastritis, gastritis varialoforme, ulcerative colitis, coeliac disease, regional ileitis, irritable bowel syndrome, etc.), gingivitis, inflammation, pain and tumescence after operation or injury, pyrexia, pain and other conditions associated with inflammation, allergic disease, systemic lupus crythematosus, scleroderma, polymyositis, tendinitis, bursitis, periarteritis nodose, rheumatic fever, Sjgren's syndrome, Behcet disease, thyroiditis, type I diabetes, diabetic complication (diabetic microangiopathy, diabetic retinopathy, diabetic neohropathy, etc.), nephrotic syndrome, aplastic anemia, myasthenia gravis, uveitis contact dermatitis, psoriasis, Kawasaki disease, sarcoidosis, Hodgkin's disease, Alzheimers disease, kidney dysfunction (nephritis, nephritic syndrome, etc.), liver dysfunction (hepatitis, cirrhosis, etc.), gastrointestinal dysfunction (diarrhea, inflammatory bowel disease, etc.) shock, bone disease characterized by abnormal bone metabolism such as osteoporosis (especially, postmenopausal osteoporosis), hypercalcemia, hyperparathyroidism, Paget's bone diseases, osteolysis, hypercalcemia of malignancy with or without bone metastases, rheumatoid arthritis, periodonritis, osteoarthritis, ostealgia, osteopenia, cancer cachexia, calculosis, lithiasis (especially, urolithiasis), solid carcinoma, mesangial proliferative glomerulonephritis, edema (e.g. cardiac edema, cerebral edema, etc.), hypertension such as malignant hypertension, premenstrual tension, urinary calculus, oliguria such as the one caused by acute or chronic failure, hyperphosphaturia."

United State Patent No 6,710,072 teaches the use of EP2 agonists for the treatment or prevention of "osteoporosis, constipation, renal disorders, sexual dysfunction, baldness, diabetes, cancer and in disorder of immune regulation...various pathophysiological diseases including acute myocardial infarction, vascular thrombosis, hypertension, pulmonary hypertension, ischemic heart disease, congestive heart failure, and angina pectoris."

These compounds may also be used to treat or prevent conditions affecting the posterior part of the eye including maculopathies/ retinal degeneration such as non-exudative age related macular degeneration (ARMD), exudative age related macular degeneration (ARMD), choroidal neovascularization, diabetic retinopathy, acute macular neuroretinopathy, central serous chorioretinopathy, cystoid macular edema, and diabetic macular edema; uveitis/ retinitis/ choroiditis such as acute multifocal placoid pigment epitheliopathy, Behcet's disease, birdshot retinochoroidopathy, infectious (syphilis, lyme, tuberculosis, toxoplasmosis), intermediate uveitis (pars planitis), multifocal choroiditis, multiple evanescent white dot syndrome (mewds), ocular sarcoidosis, posterior scleritis, serpiginous choroiditis, subretinal fibrosis and uveitis syndrome, Vogt-Koyanagi-and Harada syndrome; vasuclar diseases/ exudative diseases such as retinal arterial occlusive disease, central retinal vein occlusion, disseminated intravascular coagulopathy, branch retinal vein occlusion, hypertensive fundus changes, ocular ischemic syndrome, retinal arterial microaneurysms, Coat's disease, parafoveal telangiectasis, hemi-retinal vein occlusion, papillophlebitis, central retinal artery occlusion, branch retinal artery occlusion, carotid artery disease (CAD), frosted branch angiitis, sickle cell retinopathy and other hemoglobinopathies, angioid streaks, familial exudative vitreoretinopathy, and Eales disease; traumatic/ surgical conditions such as sympathetic ophthalmia, uveitic retinal disease, retinal detachment, trauma, conditions caused by laser, conditions caused by photodynamic therapy, photocoagulation, hypoperfusion during surgery, radiation retinopathy, and bone marrow transplant retinopathy; proliferative disorders such as proliferative vitreal retinopathy and epiretinal membranes, and proliferative diabetic retinopathy; infectious disorders such as ocular histoplasmosis, ocular toxocariasis, presumed ocular histoplasmosis syndrome (POHS), endophthalmitis, toxoplasmosis, retinal diseases associated with HIV infection, choroidal disease associate with HIV infection, uveitic disease associate with HIV infection, viral retinitis, acute retinal necrosis, progressive outer retinal necrosis, fungal retinal diseases, ocular syphilis, ocular tuberculosis, diffuse unilateral subacute neuroretinitis, and myiasis; genetic disorders such as retinitis pigmentosa, systemic disorders with accosiated retinal dystrophies, congenital stationary night blindness, cone dystrophies, Stargardt's disease and fundus flavimaculatus, Best's disease, pattern dystrophy of the retinal pigmented epithelium, X-linked retinoschisis, Sorsby's fundus dystrophy, benign concentric maculopathy, Bietti's crystalline dystrophy, and pseudoxanthoma elasticum; retinal tears/ holes such as retinal detachment, macular hole, and giant retinal tear; tumors such as retinal disease associated with tumors, congenital hypertrophy of the retinal pigmented epithelium, posterior uveal melanoma, choroidal hemangioma, choroidal osteoma, choroidal metastasis, combined hamartoma of the retina and retinal pigmented epithelium, retinoblastoma, vasoproliferative tumors of the ocular fundus, retinal astrocytoma, and intraocular lymphoid tumors; and miscellaneous other diseases affecting the posterior part of the eye such as punctate inner choroidopathy, acute posterior multifocal placoid pigment epitheliopathy, myopic retinal degeneration, and acute retinal pigement epitheliitis. Preferably, the disease or condition is retinitis pigmentosa, proliferative vitreal retinopathy (PVR), age-related macular degeneration (ARMD), diabetic retinopathy, diabetic macular edema, retinal detachment, retinal tear, uveitus, or cytomegalovirus retinitis.

These compounds may also be useful in treating asthma.

The process shown in Scheme 1 does not form part of the invention. 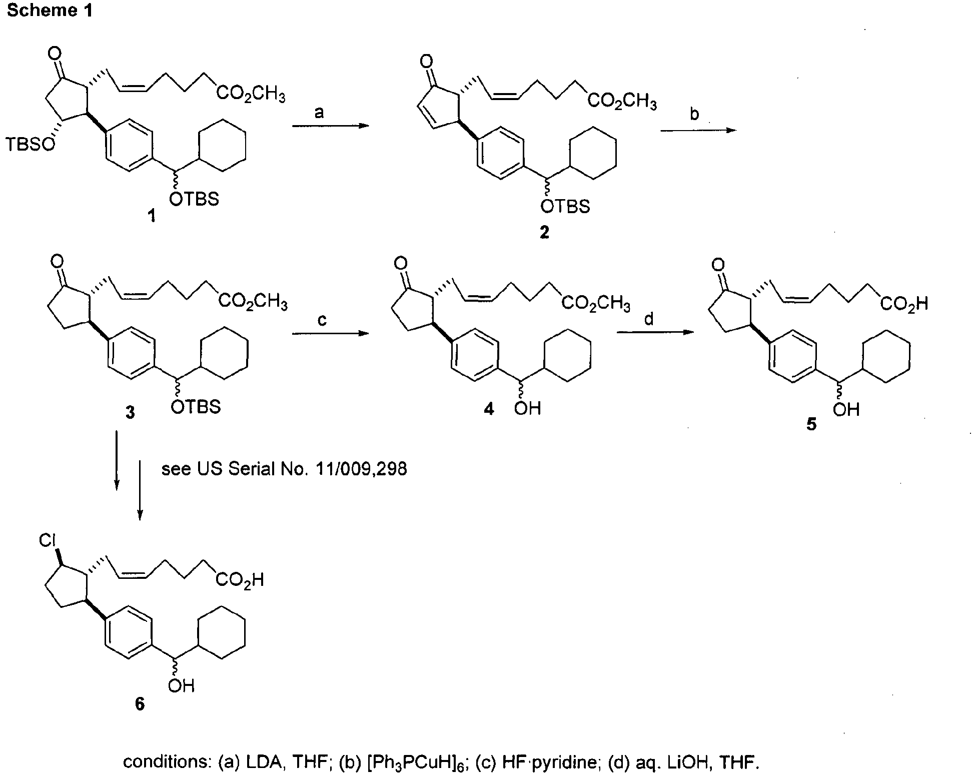 Compounds appearing in entries 1-21 in table 1 were prepared in an analogous method to that described below and as shown in scheme 1. Preparation of intermediates such as compound 1 (scheme 1) is described in United States Patent Application Serial No. 11/009,298, filed December 10, 2004.
**(Z)-7-((1R,3R)-3-(*tert***-**Butyl-dimethyl-silanyloxy)-2-{4-[(*tert*-butyl-dimethyl-silanyloxy)-cyclohexyl-methyl]-phenyl}-5-oxo-cyclopentyl)-hept-5-enoic acid methyl ester (1).** The synthesis of 1 was described in United States Patent Application Serial No. 11/009,298.
(**Z**)**-7-((R)-2-{4**-[**(*tert*-Butyl-dimethyl-silanyloxy)-cyclohexyl-methyl]-phenyl}-5-oxo-cyclopent-3**-**enyl)-hept-5-enoic acid methyl ester (2).** A -78 ° THF (50 mL) solution of **1** (176 mg, 0.27 mmol) was treated with LDA (0.67 mL, 1.34 mmol, 2M in heptane/THF/ethylbenzene). The reaction was stirred for 2 h at -78 °C and for 2 h at room temperature. Saturated NH₄Cl solution was then added and the resulting mixture was extracted with dichloromethane (3x). The combined dichloromethane solution was then dried (Na₂SO₄), filtered and evaporated. The residue was purified by flash chromatography on silica gel (10% ethyl acetate/hexanes) which gave **2** (76 mg, 54%).
**(Z)-7-((1R**,**2S)-2-{4-[(*tert*-Butyl-dimethyl-silanyloxy)-cyclohexyl**-**methyl]-phenyl}-5-oxo-cyclopentyl)-hept**-**5**-**enoic acid methyl ester (3).** A solution of **2** (76 mg, 0.15 mmol) in toluene (1 mL) was added, by cannula, to a -40 °C mixture of [Ph₃PCuH]₆ (150 mg, 0.077 mmol) in toluene (6 mL), rinsing with 0.5 mL toluene. The mixture was stirred for 1 h at -40 °C and then at room temperature overnight. The reaction was then quenched by addition of saturated NH₄Cl solution and the resulting mixture was extracted with ethyl acetate (3X). The combined organic solution was then dried (Na₂SO₄), filtered and evaporated. Purification by flash chromatography (8% ethyl acetate/hexanes) gave **3** (72 mg, 94%).
**(Z)-7-{(1R,2S)-2-[4-(Cyclohexyl-hydroxy-methyl)-phenyl]-5-oxo-cyclopentyl}-hept-5-enoic acid methyl ester (4).** HF·pyridine (800 µL) was added to an ice-cold solution of **3** (72 mg, 0.14 mmol) in CH₃CN. The reaction was allowed to stir for 1 h and then was quenched by addition of saturated NaHCO₃ solution. The resulting mixture was extracted with dichloromethane (3 x 30 mL) and the combined dichloromethane solution was dried (Na₂SO₄), filtered and evaporated. Flash chromatography on silica gel (20% ethyl acetate/hexanes) gave **4** (47 mg, 83%).
**(Z)-7-{(1R,2S)-2-[4-(Cyclohexyl-hydroxy-methyl)-phenyl]-5-oxo-cyclopentyl}-hept-5-enoic acid (5).** LiOH (1 mL, 1 mmol, 1 M) was added to a solution of **4** (43 mg, 0.10 mmol) in THF (5.5 mL). The mixture was stirred overnight and then 1 M HCl was added. The resulting mixture was extracted with dichloromethane (3 x 25 mL) and the combined organic solution was dried (Na₂SO₄), filtered and evaporated. Flash chromatography on silica gel (3% methanol/dichloromethane) gave **5** (41 mg, 100%).
**(Z)-7-{(1R,2R,5S)-2-Chloro-5-[4-(cyclohexyl-hydroxy-methyl)-phenyl]-cyclopentyl}-hept-5-enoic acid methyl ester (6).** The C9 ketone was converted to the C9 chloride using a similar procedure to the one described in United States Patent Application Serial No. 11/009,298. 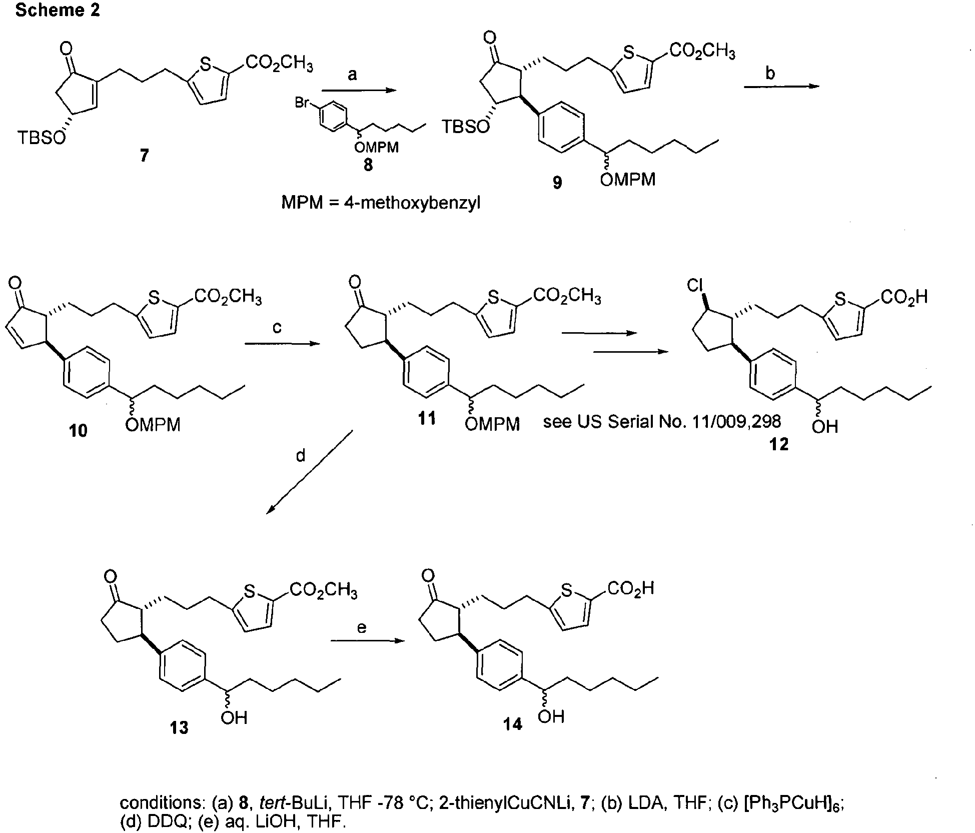 **5-{3-[(R)-3-(*tert*-Butyl-dimethyl-silanyloxy)-5-oxo-cyclopent-1-enyl]-propyl}-thiophene-2-carboxylic acid methyl ester (7).** Compound **7** was prepared as described for compound **1-8** in U.S. Provisional Patent Application No. 60/742,779, filed December 10, 2005 (scheme 1).
**1-(4-Bromo-phenyl)-hexan-1-ol 4-methoxybenzyl ether (8).** Compound **8** was prepared by protection of the corresponding alcohol (compound **1-4,** U.S. Provisional Patent Application No. 60/742,779) with 4-methoxybenzyl chloride using the procedure described in United States Patent Application Serial No. 11/009,298 for compound **7-2.**
**5-[3-((1R,2S,3R)-3-(*tert*-Butyl-dimethyl-silanyloxy)-2-{4-[1-(4-methoxy-benzyloxy)-hexyl]-phenyl}-5-oxo-cyclopentyl)-propyl]-thiophene-2-carboxylic acid methyl ester (9).** Compound **9** was prepared using the general 2-component coupling procedure described in United States Patent Application Serial No. 11/009,298.
**5-[3-((1R,2S)-2-{4-[1-(4-Methoxy-benzyloxy)-hexyl]-phenyl}-5-oxo-cyclopent-3-enyl)-propyl]-thiophene-2-carboxylic acid methyl ester (10).** Compound **10** was prepared using a similar procedure to that described for 2.
**5-[3-((1R,2S)-2-{4-[1-(4-Methoxy-benzyloxy)-hexyl]-phenyl}-5-oxo-cyclopentyl)-propyl]-thiophene-2-carboxylic acid methyl ester (11).** Compound **11** was prepared using a similar procedure to that described for 3.
**5-(3-{(1R,2R,5S)-2-Chloro-5-[4-(1-hydroxy-hexyl)-phenyl]-cyclopentyl}-propyl)-thiophene-2-carboxylic acid (12).** Compound **12** was prepared using a sequence similar to that described in United States Patent Application Serial No. 11/009,298.
**5-(3-{(1R,2S)-2-[4-(1-Hydroxy-hexyl)-phenyl]-5-oxo-cyclopentyl}-propyl)-thiophene-2-carboxylic acid methyl ester (13).** Compound **13** was prepared using the general DDQ deprotection procedure described in United States Patent Application Serial No. 11/009,298.
**5-(3-{(1R,2S)-2-[4-(1-Hydroxy-hexyl)-phenyl]-5-oxo-cyclopentyl}-propyl)-thiophene-2-carboxylic acid (14).** Compound **14** was prepared using the general LiOH procedure described in United States Patent Application Serial No. 11/009,298. 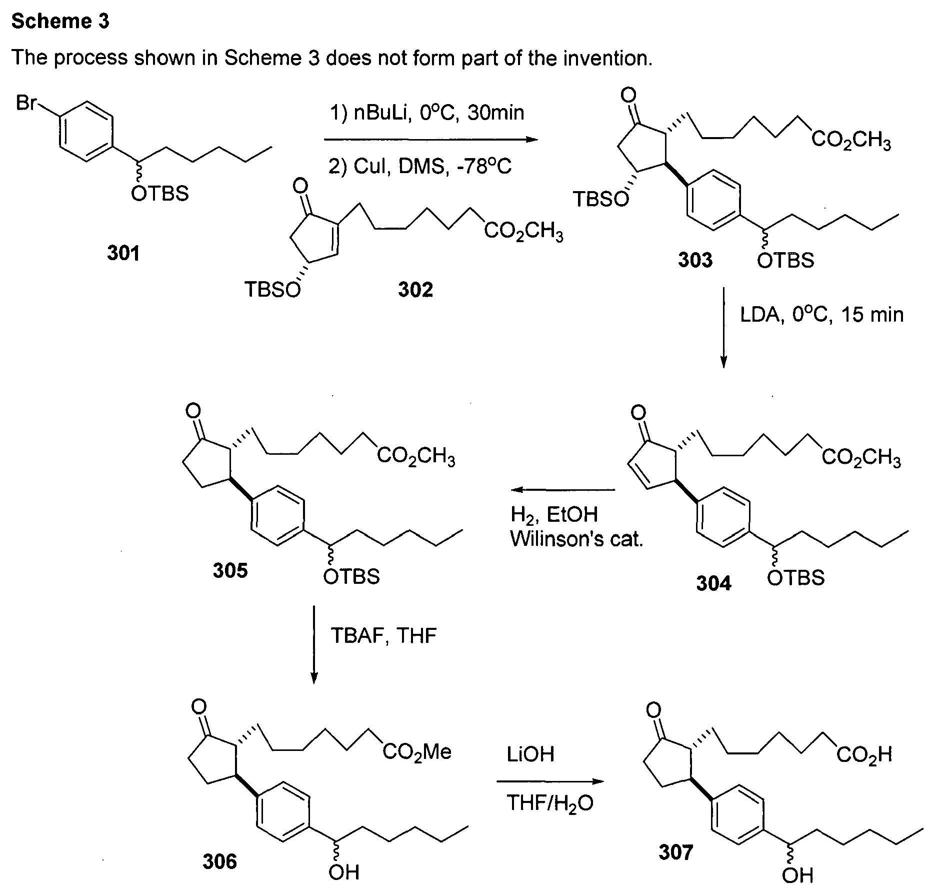
Compound 303. nBuLi (3.37mL, 5.4mmol) was added to a solution of 301 (2.0g, 5.4mmol) in ether (10mL) at 0°C. After 30 min at 0°C, the mixture was cooled to -78°C, a solution of Cul (1.0g, 5.27mmol) in DMS (2.5mL) was added, and the mixture turned yellow to orange to dark brown within 5 min and the mixture was slowly warmed to -30°C after 50min at which point the reaction mixture became almost black solution. A solution of **302** (1.84g, 5.2mmol) in DMS/ether (3/3mL) was added dropwise at -78°C and stirred for 40min at the temp. The reaction mixture was then warmed to -30°C and stirred for 16hrs (the mixture had a yellow ppt). NH₄Cl (sat.) was added and stirred for 30 min. The blue mixture was concentrated under reduced pressure. The crude was diluted with NH₄Cl (sat) and extracted with ether (x3). The combined organics were washed with brine, dried (Na₂SO₄), concentrated & purified by FCC (9:1 hex: ethyl acetate) to give 1.82g of product.
**Compound 304.** LDA (1.88mL, 2.82 mmol; 1.5M) was added to a solution of **303** (1.82g, 2.82mmol) in THF (40mL) at 0°C. After stirring for 20min at 0°C, the reaction mixture was quenched with NH₄Cl (sat) and concentrated. The crude was diluted with NH₄Cl (sat) and extracted with ethyl acetate (3x). The combined organics were washed with brine, dried (Na₂SO₄), concentrated and purified by FCC (100% hex -> 9:1 hex: ethyl acetate) to give 844.5 mg.
**Compound 305.** Wilkinson's catalyst (291 mg, 0.318mmol) was added to a solution of **304** (844mg, 1.64mmol) in EtOH (20mL) at room temperature. The reaction mixture was charged with H₂ gas and stirred for 16 hrs. The mixture was then concentrated and purified by FCC (100% hex -> 9.4:0.6 hex: ethyl acetate) to give 735 mg product.
**Compound 306.** TBAF (2.76 mL, 2.76 mmol) was added to a solution of **305** (735mg, 1.42mmol) in THF (40mL) at room temperature. The mixture was stirred for 16h and concentrated. The crude was diluted with brine and extracted with ethyl acetate (3x). The combined organics were washed with brine, dried (Na₂SO₄), concentrated & purified by FCC (3:1 hex: ethyl acetate) to give 486 mg of product.
**Compound 307.** LiOH (2.8 mg, 0.066 mmol) was added to a solution of **306** (14mg, 0.035 mmol) in THF/H₂O (2/1 mL). The mixture was stirred for 16h at room temperature and concentrated. The crude was purified by FCC (3:2 hex: ethyl acetate) to give 4.1 mg of product. 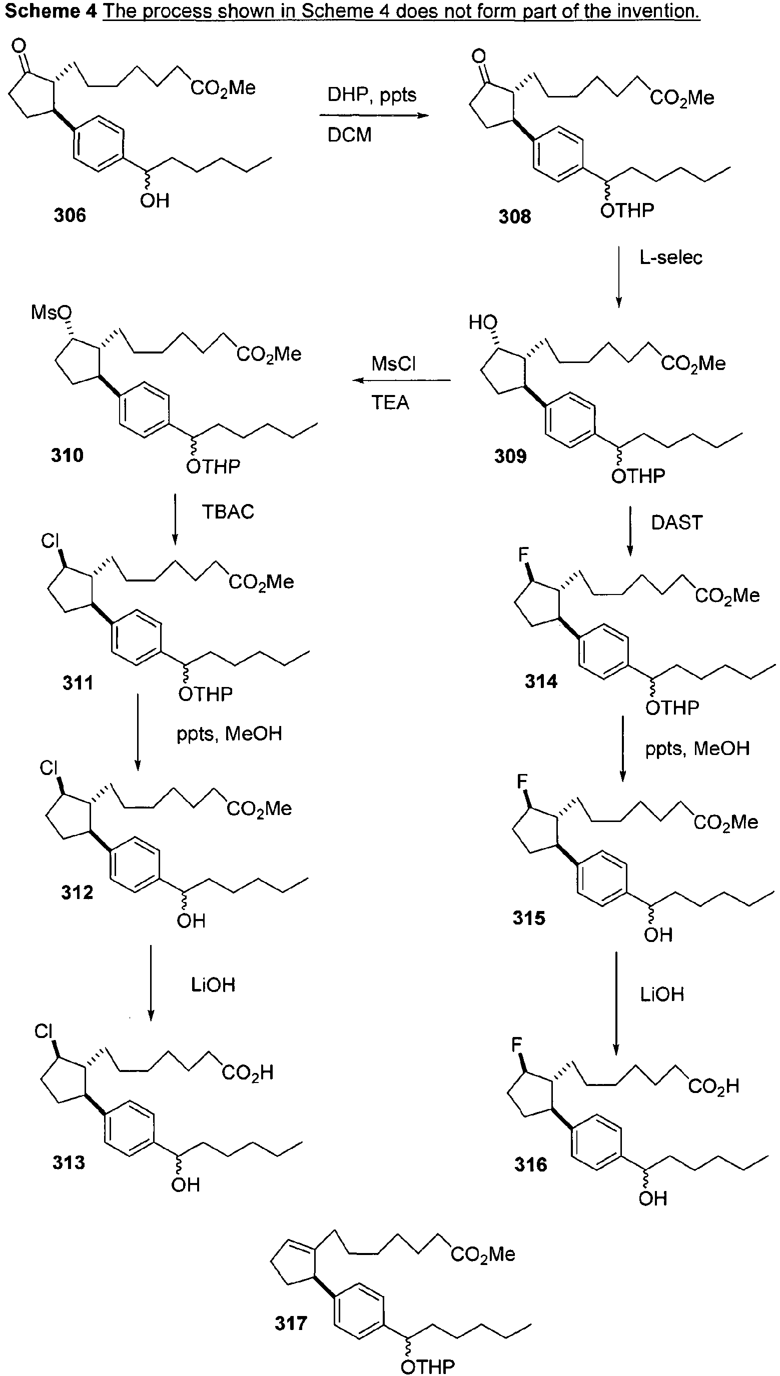
**Compound 308.** DHP (780mg, 9.28mmol) was added to a mixture of **306** (486mg, 1.27 mmol) and ppts (97mg, 0.38mmol) in DCM (20mL). After 16h at room temperature, the mixture was concentrated and purified by FCC (100% hex -> 7 :1 hex : ethyl acetate) to give 535 mg of product.
**Compound 309.** L-selectride (687mmol, 687 microL) was added to a solution of **308** (220mg, 0.45 mmol) in THF (10mL) at - 78°C. After 30min, H₂O₂ (430 microL) was added and the mixture was warmed to 0°C. After 20min at 0°C, the mixture was then quenched with 2N HCl (430microL) and concentrated. The crude was diluted with brine and extracted with ethyl acetate (3x). The combined organics were washed with brine, dried (Na₂SO₄), concentrated and purified by FCC (100% hex -> 4:1 hex: ethyl acetate) to give 160mg product.
**Compound 310.** MsCl (22.7 microL x 2, 0.288 mmol x 2) was added to a mixture of **309** (116mg, 0.24mmol) and TEA (50microL x 2, 0.36 mmol x 2) in DCM (5mL) at room temperature. After 1 h, the mixture became a yellow solution and TLC indicated no change; the mixture was allowed to stir for 16h. The mixture was quenched with NaHCO₃ (sat) and washed with ethyl acetate (2x) + hexanes (1 x). The combined organics were washed with brine, dried (Na₂SO₄) and concentrated. FCC (4:1 hex: ethyl acetate) gave 96 mg product.
**Compound 311.** TBAC (420mg, 1.51mmol) was added to a solution of **310** (86mg, 0.151mmol) in toluene (5mL). The mixture was warmed to 40°C and stirred for 16h. The mixture was diluted with water & brine, and extracted with ethyl acetate (3x). The combined organics were washed with brine, dried (Na₂SO₄), concentrated and purified by FCC (100% hex -> 9:1 hex: ethyl acetate) to give 47 mg product.
**Compound 312.** Ether **311** (45mg, 0.089mmol) and ppts (22.3 mg, 0.084mmol) were mixed & stirred in MeOH (5mL) for 16h. The mixture was concentrated and purified by FCC (9:1 -> 7:1 hex: ethyl acetate) to give 27mg of product.
**Compound 313.** LiOH (5mg, 0.118mmol) was added to a solution of ester (25mg, 0.059mmol) in THF/H₂O (2/2mL). After 16h, the mixture was concentrated & purified by FCC (3:2 hex: ethyl acetate) to give 16.6 mg of product.
**Compound 314.** DAST (26.4mg, 0.164mmol) was added to a solution of **309** (40mg, 0.0819 mmol) in DCM (5mL) at -78°C. After 20min at -78°C the mixture was quenched with NaHCO₃ (aq) & warmed to room temperature. The mixture was extracted with DCM (3x) and the combined organics were washed with brine, dried (Na₂SO₄), concentrated & purified by prep TLC to give 16mg **317** & 15.6mg **314.** 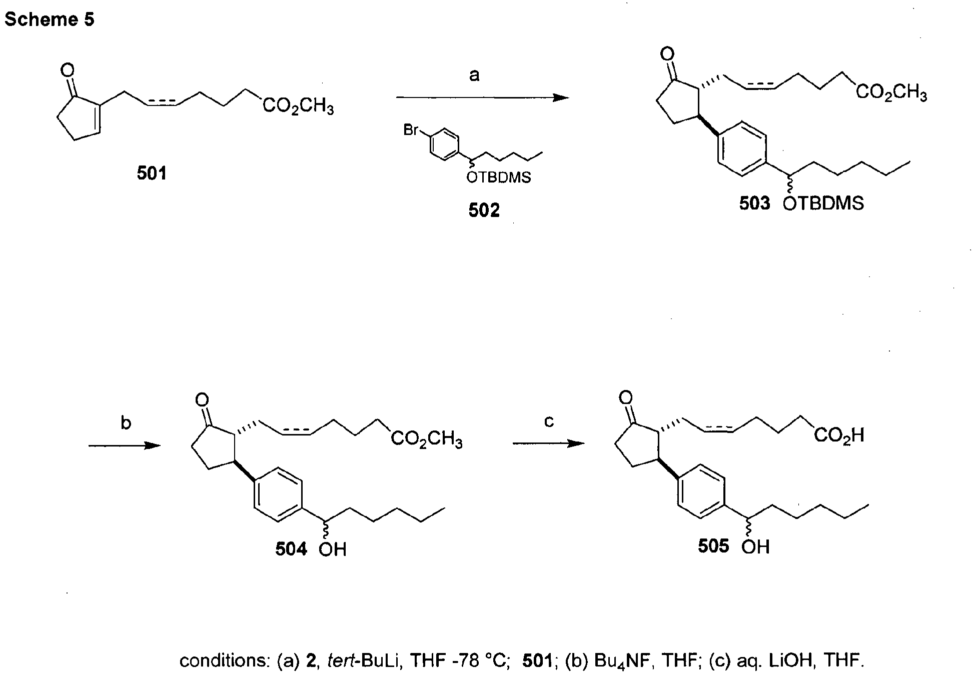
The compounds of scheme 5 were prepared using procedures similar to those disclosed above. The process shown in Scheme 5 does not form part of the invention.

### Binding Data

### Ki

Competition binding experiments were performed in a medium containing Hank's balanced salt solution, Hepes 20 mM, pH 7.3, membranes (∼60 µg protein) or 2x10⁵ cells from HEK 293 cells stably expressing human EP2 receptors, [³H]PGE2 (10 nM) and various concentrations of test compounds in a total volume of 300 µl. Reaction mixtures were incubated at 23 °C for 60 min, and were filtered over Whatman GF/B filters under vacuum. Filters were washed three times with 5 ml ice-cold buffer containing 50 mM Tris/HCl (pH 7.3). Non-specific binding was estimated in the presence of excess unlabeled PGE2 (10 µM). Binding data fitted to the binding model for a single class of binding sites, using nonlinear regression analysis. IC₅₀ values thus obtained were converted to Ki using the equation of Ki=(IC₅₀/(1+[L]/K_{D}) where [L] represents PGE2 concentration (10 nM) and K_{D} the dissociation constant for [³H]PGE2 at human EP2 receptors (40 nM).

### Radioligand Binding

### Cells Stably Expressing EP₁, EP₂, EP₄ and FP Receptors

HEK-293 cells stably expressing the human or feline FP receptor, or EP₁, EP₂, or EP₄ receptors were washed with TME buffer, scraped from the bottom of the flasks, and homogenized for 30 sec using a Brinkman PT 10/35 polytron. TME buffer was added to achieve a final 40 ml volume in the centrifuge tubes (the composition of TME is 100 mM TRIS base, 20 mM MgCl₂, 2M EDTA; 10N HCl is added to achieve a pH of 7.4).

The cell homogenate was centrifuged at 19000 r.p.m. for 20 min at 4° C using a Beckman Ti-60 rotor. The resultant pellet was resuspended in TME buffer to give a final 1 mg/ml protein concentration, as determined by Biorad assay. Radioligand binding competition assays vs. [³H-]17 -phenyl PGF_{2_{α}} (5 nM) were performed in a 100µl volume for 60 min. Binding reactions were started by adding plasma membrane fraction. The reaction was terminated by the addition of 4 ml ice-cold TRIS-HCl buffer and rapid filtration through glass fiber GF/B filters using a Brandel cell harvester. The filters were washed 3 times with ice-cold buffer and oven dried for one hour.

[³H-] PGE₂ (specific activity 180 Ci mmol) was used as the radioligand for EP receptors. [³H] 17-phenyl PGF_{2_{α}} was employed for FP receptor binding studies. Binding studies employing EP₁, EP₂, EP₄ and FP receptors were performed in duplicate in at least three separate experiments. A 200µl assay volume was used. Incubations were for 60 min at 25°C and were terminated by the addition of 4 ml of ice-cold 50 mM TRIS-HCl, followed by rapid filtration through Whatman GF/B filters and three additional 4 ml washes in a cell harvester (Brandel). Competition studies were performed using a final concentration of 5 nM [³H]-PGE₂, or 5 nM [³H] 17-phenyl PGF_{2_{α}} and non-specific binding determined with 10⁻⁵M of unlabeled PGE₂, or 17-phenyl PGF_{2_{α}}, according to receptor subtype studied.

### METHODS FOR FLIPR™ STUDIES

### (a) CELL CULTURE

HEK-293(EBNA) cells, stably expressing one type or subtype of recombinant human prostaglandin receptors (prostaglandin receptors expressed: hDP/Gqs5; hEP₁; hEP₂/Gqs5; hEP_{3A}/Gqi5; hEP₄/Gqs5; hFP; hIP; hTP), were cultured in 100 mm culture dishes in high-glucose DMEM medium containing 10% fetal bovine serum, 2 mM I-glutamine, 250 □g/ml geneticin (G418) and 200 µg/ml hygromycin B as selection markers, and 100 units/ml penicillin G, 100 □g/ml streptomycin and 0.25 µg/ml amphotericin B.

### (b) CALCIUM SIGNAL STUDIES ON THE FLIPR™

Cells were seeded at a density of 5x10⁴ cells per well in Biocoat® Poly-D-lysine-coated black-wall, clear-bottom 96-well plates (Becton-Dickinson) and allowed to attach overnight in an incubator at 37 °C. Cells were then washed two times with HBSS-HEPES buffer (Hanks Balanced Salt Solution without bicarbonate and phenol red, 20 mM HEPES, pH 7.4) using a Denley Cellwash plate washer (Labsystems). After 45 minutes of dye-loading in the dark, using the calcium-sensitive dye Fluo-4 AM at a final concentration of 2 µM, plates were washed four times with HBSS-HEPES buffer to remove excess dye leaving 100 µl in each well. Plates were re-equilibrated to 37 °C for a few minutes.

Cells were excited with an Argon laser at 488 nm, and emission was measured through a 510-570 nm bandwidth emission filter (FLIPR™, Molecular Devices, Sunnyvale, CA). Drug solution was added in a 50 □l volume to each well to give the desired final concentration. The peak increase in fluorescence intensity was recorded for each well. On each plate, four wells each served as negative (HBSS-HEPES buffer) and positive controls (standard agonists: BW245C (hDP); PGE₂ (hEP₁; hEP₂/Gqs5; hEP_{3A}/Gqi5; hEP₄/Gqs5); PGF_{2_{α}} (hFP); carbacyclin (hIP); U-46619 (hTP), depending on receptor). The peak fluorescence change in each drug-containing well was then expressed relative to the controls.

Compounds were tested in a high-throughput (HTS) or concentration-response (CoRe) format. In the HTS format, forty-four compounds per plate were examined in duplicates at a concentration of 10⁻⁵ M. To generate concentration-response curves, four compounds per plate were tested in duplicates in a concentration range between 10⁻⁵ and 10⁻¹¹ M. The duplicate values were averaged. In either, HTS or CoRe format each compound was tested on at least 3 separate plates using cells from different passages to give an n ≥ 3.

The results of the binding and activity studies, presented in Tables 1-3 demonstrate that the compounds disclosed herein are selective prostaglandin EP₂ agonists, and are thus useful for the treatment of glaucoma, ocular hypertension, the other diseases or conditions disclosed herein.

**Table 1. Entries 1 - 21 do not form part of the invention.**

| | | **BINDING (Kᵢ, nM)** | | | | | | **FUNCTIONAL (Ca2+, EC50, nM)** | | | | | |
|---|---|---|---|---|---|---|---|---|---|---|---|---|---|
| **Entry** | **STRUCTURE** | **HEP 2** | **HEP 3** | **HEP 4** | **HFP** | **HEP 1** | **HEP 2** | | **HEP 3A** | **HEP 4** | **HTP** | **HIP** | **HDP** |
| 1 | 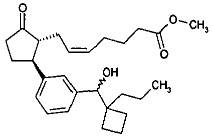 | | | | **NA** | **NA** | **NA** | | **NA** | **NA** | **>10 K** | **NA** | **NA** |
| 2 | 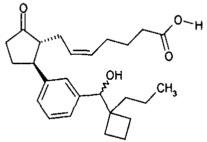 | | | | **NA** | **NA** | **2657** | | **2789** | **NA** | **NA** | **NA** | **NA** |
| 3 | 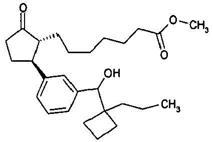 | | | | **NA** | **NA** | **NA** | | **NA** | **NA** | **NA** | **NA** | **NA** |
| 4 | 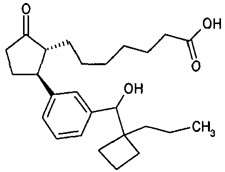 | | | | **NA** | **NA** | **3407** | | **3828** | **NA** | **NA** | **NA** | **NA** |
| 5 | 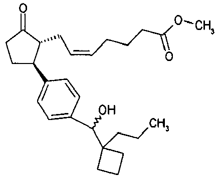 | | | | **NA** | **NA** | **NA** | | **NA** | **NA** | **NA** | **NA** | **NA** |
| 6 | 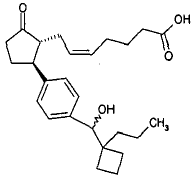 | | | | **NA** | **NA** | **2872** | | **NA** | **NA** | **NA** | **NA** | **>10 K** |
| 7 | 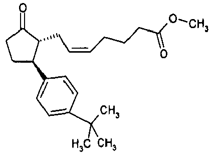 | | | | **NA** | **NA** | **NA** | | **NA** | **NA** | **NA** | **NA** | **NA** |
| 8 | 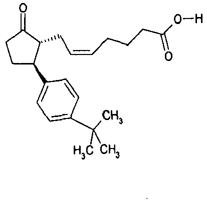 | | | | **NA** | **NA** | **1050** | | **NA** | **NA** | **NA** | **NA** | **NA** |
| 9 | 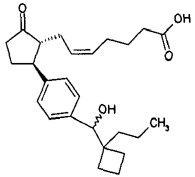 | | | | **NA** | **NA** | **>10 K** | | **NA** | **>10 K** | **NA** | **NA** | **NA** |
| 10 | 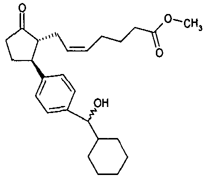 | | | | **NA** | **NA** | **>10 K** | | **NA** | **NA** | **NA** | **NA** | **NA** |
| 11 | 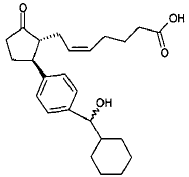 | | | | **NA** | **NA** | **1640** | | **NA** | **NA** | **NA** | **NA** | **NA** |
| 12 | 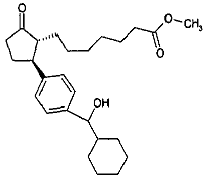 | | | | **NA** | **NA** | **>10 K** | | **NA** | **NA** | **>10 K** | **NA** | **NA** |
| 13 | 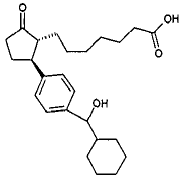 | | | | **NA** | **NA** | **2047** | | **NA** | **NA** | **NA** | **NA** | **NA** |
| 14 | 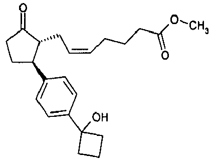 | | | | **NA** | **NA** | **NA** | | **NA** | **NA** | **NA** | **NA** | **NA** |
| 15 | 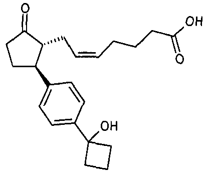 | | | | **NA** | **NA** | **NA** | | **NA** | **NA** | **NA** | **NA** | **NA** |
| 16 | 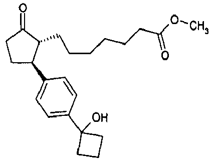 | | | | **NA** | **NA** | **NA** | | **NA** | **NA** | **NA** | **NA** | **NA** |
| 17 | 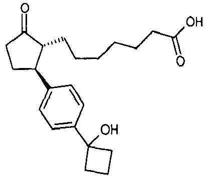 | | | | **NA** | **NA** | **>10 K** | | **NA** | **NA** | **NA** | **NA** | **NA** |
| 18 | 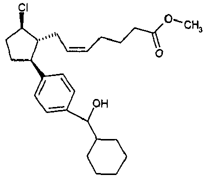 | | | | **NA** | **NA** | **NA** | | **NA** | **NA** | **NA** | **NA** | **NA** |
| 19 | 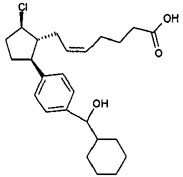 | | | | **NA** | **NA** | **2101** | | **NA** | **NA** | **NA** | **NA** | **NA** |
| 20 | 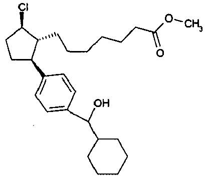 | | | | **NA** | **NA** | **NA** | | **NA** | **NA** | **NA** | **NA** | **NA** |
| 21 | 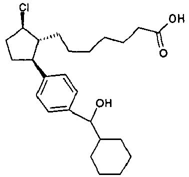 | | | | **NA** | **NA** | **>10 K** | | **NA** | **NA** | **>10 K** | **NA** | **NA** |
| 22 | 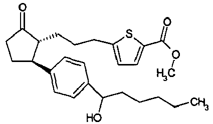 | | | | | | | | | | | | |
| 23 | 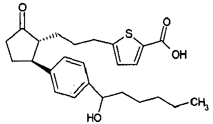 | **54** | | | **NA** | **NA** | **2 (3)^{a}** | | **55** | **>10 K** | **NA** | **NA** | **NA** |
| 24 | 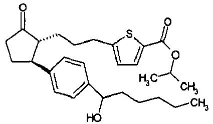 | | | | | | | | | | | | |
| 25 | 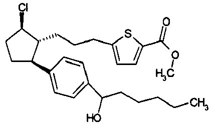 | | | | | | | | | | | | |
| 26 | 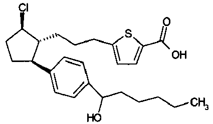 | | | | | | | | | | | | |

| | | | | | | | | | | | | | |
|---|---|---|---|---|---|---|---|---|---|---|---|---|---|
| (a) Refers to EC₅₀ in a cAMP mediated assay NA means "not active." | | | | | | | | | | | | | |

**Table 2. The structures of Table 2 do not form part of the invention.**

| STRUCTURE | BINDING HEP2 | FUNCTIONAL HEP2 |
|---|---|---|
| 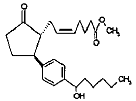 | 15000 | 15000 |
| 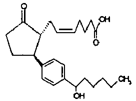 | 700 | 1011 |
| 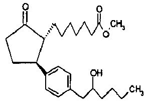 | 4600 | 15000 |
| 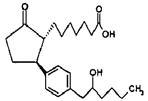 | 700 | 343 |
| 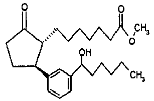 | 50000 | 50000 |
| 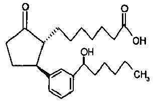 | 15000 | 50000 |
| 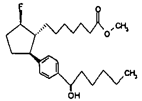 | 50000 | 50000 |
| 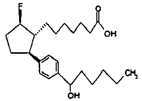 | 600 | 15000 |
| 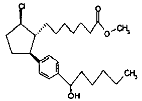 | 50000 | 50000 |
| 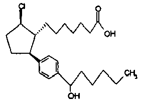 | 1900 | 15000 |
| 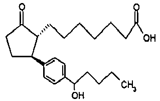 | 1400 | 1409 |
| 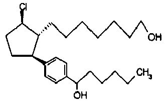 | 50000 | 50000 |
| 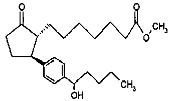 | 15000 | 15000 |
| 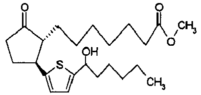 | 7100 | 7100 |
| 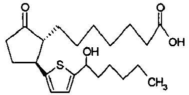 | 600 | 769 |
| 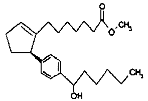 | 50000 | 50000 |
| 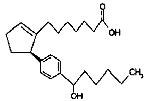 | 2811 | 4877 |

**Table 3 The structures of Table 3 do not form part of the invention.**

| | **BINDING (Ki, nM)** | | | **FUNCTIONAL (Ca2+, EC50, nM)** | | | | | | | |
|---|---|---|---|---|---|---|---|---|---|---|---|
| **STRUCTURE** | **HEP2** | **HEP3** | **HEP4** | **HFP** | **HEP1** | **HEP2** | **HEP3A** | **HEP4** | **HTP** | **HIP** | **HDP** |
| 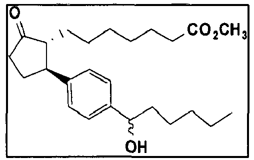 | 4677 | NA | 15000 | NA | NA | 2162 | 3090 | NA | NA | NA | NA |
| 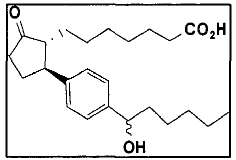 | 871 | 5200 | 15000 | NA | NA | 284 | 90 | NA | NA | NA | NA |
| 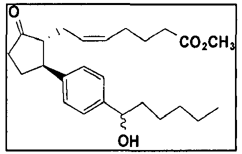 | 15000 | | 15000 | NA | NA | 15000 | 15000 | NA | NA | NA | NA |
| 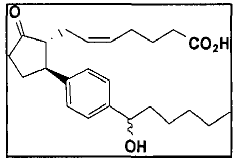 | 700 | 15000 | 15000 | NA | NA | 1011 | 552 | NA | NA | NA | NA |
| (a) Refers to EC₅₀ in a cAMP mediated assay | | | | | | | | | | | |
| NA means "not active." | | | | | | | | | | | |

Treatment of inflammatory bowel disease may be accomplished by the administration of the compounds described herein to the suffering mammal. Inflammatory bowel disease describes a variety of diseases characterized by inflammation of the bowels including ulcerative colitis and Crohn's disease. Treatment may be accomplished by oral administration, by suppository, or parenteral administration, or some other suitable method.

Delivery of the compounds disclosed herein to the colon via oral dosage forms may be accomplished by any of a number of methods known in the art. For example, reviews by Chourasia and Jain in J Pharm Pharmaceut Sci 6 (1): 33-66, 2003 and Shareef et. al (AAPS PharmSci 2003; 5 (2) Article 17) describe a number of useful methods. These methods include 1) administration of a prodrug, including an azo or a carbohydrate based prodrug; 2) coating the drug with, or encapsulating or impregnating the drug into a polymer designed for delivery to the colon, 3) time released delivery of the drug, 4) use of a bioadhesive system.

While not intending to be bound in any way by theory, it is believed that intestinal microflora are capable of reductive cleavage of an azo bond leaving the two nitrogen atoms as amine functional groups. The azo prodrug approach has been used to deliver to 5-aminosalicylic acid to the colons of humans in clinical trials for the treatment of inflammatory bowel disease. It is also believed that bacteria of the lower Gl also have enzymes which can digest glycosides, glucuronides, cyclodextrins, dextrans, and other carbohydrates, and ester prodrugs formed from these carbohydrates have been shown to deliver the parent active drugs selectively to the colon. For example, in vivo and in vitro studies on rats and guinea pigs with prodrugs of dexamethasone, prednisolone, hydrocortisone, and fludrocortisone, suggest that glycoside conjugates may be useful for the delivery of steroids to the human colon. Other in vivo studies have suggested that glucouronide, cyclodextrin, and dextran prodrugs of steroids or non-steroidal anti-inflammatory drugs are useful for delivery of these drugs to the lower Gl tract. An amide of salicylic acid and glutamic acid has been shown to be useful for the delivery of salicylic acid to the colon of rabbit and dog.

Carbohydrate polymers such as amylase, arabinogalactan, chitosan, chondroiton sulfate, dextran, guar gum, pectin, xylin, or azo-group containing polymers can be used to coat a drug compound, or a drug may be impregnated or encapsulated in the polymer. It is believed that after oral administration, the polymers remain stable in the upper Gl tract, but are digested by the microflora of the lower Gl thus releasing the drug for treatment.

Polymers which are sensitive to pH may also be used since the colon has a higher pH than the upper Gl tract. Such polymers are commercially available. For example, Rohm Pharmaceuticals, Darmstadt, Germany, commercially provides pH dependent methacrylate based polymers and copolymers which have varying solubilities over different pH ranges based upon the number of free carboxylate groups in the polymer under the tradename Eudragit®. Several Eudragit® dosage forms are currently used to deliver salsalazine for the treatment of ulcerative colitis and Crohn's disease. Time release systems, bioadhesive systems, and other delivery systems have also been studied.

The foregoing description details specific methods and compositions that can be employed to practice the present invention, and represents the best mode contemplated. However, it is apparent for one of ordinary skill in the art that further compounds with the desired pharmacological properties can be prepared in an analogous manner, and that the disclosed compounds can also be obtained from different starting compounds via different chemical reactions. Similarly, different pharmaceutical compositions may be prepared and used with substantially the same result.

## Claims

1. A compound having the following structure or a pharmaceutically acceptable salt 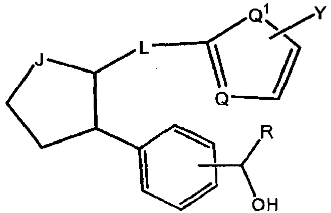 thereof:
wherein Y is an organic acid functional group, or an amide or ester thereof comprising up to 14 carbon atoms; or Y is hydroxymethyl or an ether thereof comprising up to 14 carbon atoms; or Y is a tetrazolyl functional group;
L is linear C₃H₆, C₂H₄O, or C₂H₄S;
Q is CH, S, N, or O;
Q¹ is S, N, or O;
J is C=O, CHOH, CHF, CHCl, CHBr, or CHCN; and
R is hydrogen or C₁₋₁₀ hydrocarbyl.

2. The compound according to claim 1 wherein Y is selected from CO₂R² CON(R²)₂, CON(OR²)R², CON(CH₂CH₂OH)₂, CONH(CH₂CH₂OH), CH₂OH, P(O)(OH)₂, CONHSO₂R², SO₂N(R²)₂, SO₂NHR², 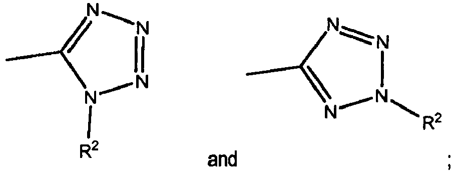 wherein R² is independently H, C₁₋C₆ alkyl, unsubstituted phenyl, or unsubstituted biphenyl.

3. The compound according to claim 1 or claim 2 wherein R is alkyl.

4. The compound according to claim 1 or claim 2 wherein R is arylalkyl.

5. The compound according to claim 1 or claim 2 wherein R is n-pentyl.

6. The compound according to any of claims 1-5 for use in the treatment of glaucoma or ocular hypertension in a mammal.

7. A kit comprising a composition comprising a compound according to any of claims 1-5, a container, and instructions for administration of said composition to a mammal for the treatment of glaucoma or ocular hypertension.

8. A composition comprising a compound according to any of claims 1-5, wherein said composition is a liquid which is ophthalmically acceptable.

## Patentansprüche

1. Verbindung mit der nachstehenden Struktur oder ein pharmazeutisch annehmbares Salz davon: 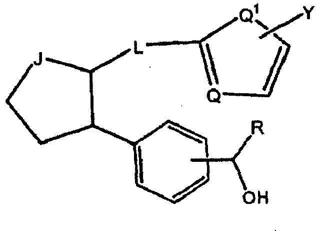 worin:
Y eine organische funktionelle Säuregruppe oder ein Amid oder Ester davon, umfassend bis zu 14 Kohlenstoffatome, ist; oder Y Hydroxymethyl oder ein Ether davon, umfassend bis zu 14 Kohlenstoffatome, ist; oder Y eine funktionelle Tetrazolylgruppe ist;
L geradkettiges C₃H₆, C₂H₄O oder C₂H₄S ist;
Q CH, S, N oder O ist;
Q¹ S, N oder O ist;
J C=O, CHOH, CHF, CHCl, CHBr oder CHCN ist; und
R Wasserstoff oder C₁₋₁₀-Hydrocarbyl ist.

2. Verbindung gemäss Anspruch 1, worin Y aus CO₂R², CON(R²)₂, CON(OR²)R², CON(CH₂CH₂OH)₂, CONH(CH₂CH₂OH), CH₂OH, P(O)(OH)₂, CONHSO₂R², SO₂N(R²)₂, SO₂NHR² 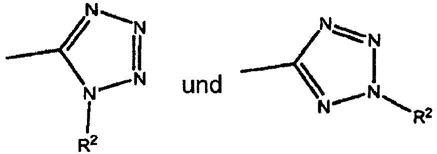 ausgewählt ist, worin R² unabhängig H, C₁₋₆-Alkyl, unsubstituiertes Phenyl oder unsubstituiertes Biphenyl ist.

3. Verbindung gemäss Anspruch 1 oder Anspruch 2, worin R Alkyl ist.

4. Verbindung gemäss Anspruch 1 oder Anspruch 2, worin R Arylalkyl ist.

5. Verbindung gemäss Anspruch 1 oder Anspruch 2, worin R n-Pentyl ist.

6. Verbindung gemäss einem der Ansprüche 1 bis 5 zur Verwendung bei der Behandlung von Glaukom oder okulärer Hypertension bei einem Säuger.

7. Kit, umfassend eine Zusammensetzung, die eine Verbindung gemäss einem der Ansprüche 1 bis 5, einen Behälter und Anweisungen zur Verabreichung dieser Zusammensetzung an einen Säuger umfasst, zur Behandlung von Glaukom und okulärer Hypertension.

8. Zusammensetzung, umfassend eine Verbindung gemäss einem der Ansprüche 1 bis 5, wobei diese Zusammensetzung eine ophthalmisch annehmbare Flüssigkeit ist.

## Revendications

1. Composé ayant la structure suivante ou un sel pharmaceutiquement acceptable de celui-ci : 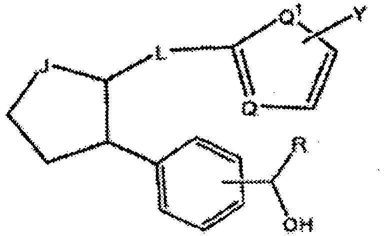
dans lequel Y est un groupe acide organique fonctionnel, ou un amide ou ester de celui-ci comprenant jusqu'à 14 atomes de carbone ; ou Y est un groupe hydroxyméthyle ou un éther de celui-ci comprenant jusqu'à 14 atomes de carbone; ou Y est un groupe fonctionnel tétrazolyle;
L est un groupe C₃H₆ linéaire, C₂H₄O, ou C₂H₄S;
Q est CH, S, N, ou O ;
Q¹ est S, N, ou O ;
J est C=O, CHOH, CHF, CHCl, CHBr, ou CHCN ; et
R représente un atome d'hydrogène ou un groupe hydrocarbyle en C₁ à C₁₀.

2. Composé selon la revendication 1 dans lequel Y est choisi parmi CO₂R², CON(R²)₂, CON(OR²)R², CON(CH₂CH₂OH)₂, CONH(CH₂CH₂OH), CH₂OH, P(O)(OH)₂, CONHSO₂R², SO₂N(R²)₂, SO₂NHR², 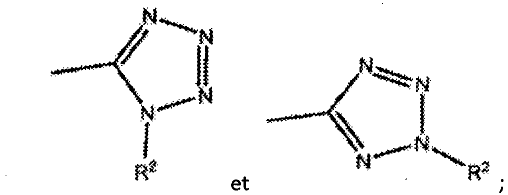 dans lequel R² est indépendamment H, un groupe alkyle en C₁ à C₆, phényle non substitué, ou biphényle non substitué.

3. Composé selon la revendication 1 ou la revendication 2, dans lequel R est un groupe alkyle.

4. Composé selon la revendication 1 ou la revendication 2, dans lequel R est un groupe arylalkyle.

5. Composé selon la revendication 1 ou la revendication 2, dans lequel R est un groupe n-pentyle.

6. Composé selon l'une quelconque des revendications 1 à 5 pour une utilisation dans le traitement du glaucome ou de l'hypertension oculaire chez un mammifère.

7. Kit comprenant une composition comprenant un composé selon l'une quelconque des revendications 1 à 5, un récipient, et des instructions pour l'administration de ladite composition à un mammifère pour le traitement du glaucome ou de l'hypertension oculaire.

8. Composition comprenant un composé selon l'une quelconque des revendications 1 à 5, dans laquelle ladite composition est un liquide qui est ophtalmiquement acceptable.
